(19) Europäisches Patentamt
European Patent Office
Office européen des brevets

(11) EP 0 344 302 B1

## (12) EUROPEAN PATENT SPECIFICATION

(45) Date of publication and mention
of the grant of the patent:
**31.03.1999 Bulletin 1999/13**

(21) Application number: **89901415.3**

(22) Date of filing: **16.11.1988**

(51) Int Cl.6: **C12N 5/04**, A01H 1/04

(86) International application number:
**PCT/US88/04116**

(87) International publication number:
**WO 89/05344 (15.06.1989 Gazette 1989/13)**

(54) **REGENERATION AND TRANSFORMATION OF COTTON**

REGENERIERUNG UND TRANSFORMATION VON BAUMWOLLE

REGENERATION ET TRANSFORMATION DU COTON

(84) Designated Contracting States:
**AT BE CH DE FR GB IT LI LU NL SE**

(30) Priority: **18.11.1987 US 122200**

(43) Date of publication of application:
**06.12.1989 Bulletin 1989/49**

(60) Divisional application: **98118057.3 / 0 899 341**

(73) Proprietor: **MYCOGEN CORPORATION
San Diego, California 92121 (US)**

(72) Inventors:
• **RANGAN, Thirumale, Srinivasa
Pasadena, CA 91107 (US)**
• **ANDERSON, David, Maurice
Altadena, CA 91001 (US)**
• **RAJASEKARAN, Kanniah
Sierra Madre, CA 91024 (US)**
• **GRULA, John, William
Pasadena, CA 91104 (US)**
• **HUDSPETH, Richard, Lorne
Altadena, CA 91001 (US)**
• **YENOFSKY, Richard, Lee
Arcadia, CA 91006 (US)**

(74) Representative: **Grünecker, Kinkeldey,
Stockmair & Schwanhäusser Anwaltssozietät
Maximilianstrasse 58
80538 München (DE)**

(56) References cited:
EP-A- 218 571          EP-A- 270 355
EP-A- 289 479          EP-A- 317 511
WO-A-86/03516          WO-A-87/02701
US-A- 4 672 035

• IN VITRO, vol. 20, 1984, page 256, abstract no. 65, 35TH ANNUAL MEETING OF THE TISSUE CULTURE ASSOCIATION, Houston, Texas, US; L.T.S. RANGAN et al.: "Somatic embryogenesis in tissue cultures of gossypium hirsutum"
• W.R. SHARP et al. (ed.): "HANDBOOK OF PLANT CELL CULTURE, vol. 2, CROPSPECIES, 1984, pages 487-510, Chapter 18, Macmillan Publishing; H.J. PRICE et al.: "Cotton"
• CHEMISTRY AND INDUSTRY, no. 7, April 1986, page 218; "Seeds of change in the outside world"
• COMMMONWEALTH AGRICULTURAL BUREAU, abstract no. CAB 861655613; J.Q. HUANG et al.: "Transfer of fusarium wilt resistance between cotton cultivars via exogenous DNA introduction" & SCIENTIA AGRICULTURA SINICA, no. 3, 1986, pages 32-36
• METHOD IN ENZYMOLOGY, vol. 101, 1983, pages 433-481, Academic Press, Inc.; G.-Y. ZHOU et al.: "Introduction of exogenous DNA into cotton embryos"
• CHEMICAL ABSTRACTS, Volume 100, No. 9, issued 1984, February 27 (Columbus, Ohio, USA), DAVIDONIS et al., "Plant Regeneration from Callus Tissue of Gossypium Hirsutum L.," see page 343, column 2, the Abstract No. 65175p, Sci. Lett. 1983, 32(1-2): 89-93 (Eng.).
• Biological Abstracts, Volume 78, No. 8, issued 15 October 1984 (Philadelphia, Pennsylvania, USA), STUART et al., "Somatic Embryogenesis from Cell Cultures of Medicago Sativa: 2: The Interaction of Amino Acids with Ammonium", see page 6339, column 1, the Abstract No. 56366, Plant Sci. Lett. 1984, 34(1/2): 175-182 (Eng.).

EP 0 344 302 B1

- TROLINDER et al., Proceedings of the Beltwide Cotton Production Research Conference, January 6-11, 1985, New Orleans, LA., page 46.
- Biological Abstracts, Volume 82, No. 9, issued 01 November 1986, (Philadelphia, Pennsylvania, USA), SHOEMAKER et al., "Characterization of Somatic Embryogenesis and Plant Regeneration in Cotton (Gossypium Hirsutum L.), "see page AB-907, colume 2, the Abstract No. 86768, Plant Cell Rep. 19868 5(3): 178-181 (Eng.).
- CHEMICAL ABSTRACTS, Volume 106, No. 9, issued 1987, March 02 (Columbus, Ohio, USA), N.L.G. TROLINDER, "Somatic Embryogenesis and Plant Regeneration in Gossypium Hirsutum L.," see page 225, column 1, the Abstract No. 62935z, Diss. Abstr. Int. B 1986, 47(6): 2250-1 (Eng.).
- CHEMICAL ABSTRACTS, Volume 107, No. 5, issued 1987, August 03 (Columbus, Ohio, USA), TROLINDER et al., "Somatic Embryogenesis and Plant Regeneration in Cotton (Gossypium Hirsutum L.)," see page 369, column 1, the Abstract No. 36159a, Plant Cell Rep. 1987, 6(3): 231-4 (Eng.).
- CHEMICAL ABSTRACTS, Volume 106, No. 21, issued 1987, May 25 (Columbus, Ohio, USA), UMBECK et al., "Genetically Transformed Cotton (Gossypium Hirsutum L.) Plants," see page 179, column 1, the Abstract No. 170182k, Bio/Technology 1987, 5(3): 263-6 (Eng.).
- CHEMICAL ABSTRACTS, Volume 108, No. 11, issued 1988, March 14 (Columbus, Ohio, USA), FIROOZABADY et al., "Transformation of Cotton (Gossypium Hirsutum L.) by Agrobacterium Tumefaciens and Regeneration of Transgenic Plants," see page 153, column 1, the Abstract No. 88857s, Plant Mol. Biol. 1987, 10(2): 105-16 (Eng.).
- The EMBO Journal, Volume 3, No. 8, issued August 1984, (Oxford, England), DE BLOCK et al., "Expression of Foreign Genes in Regenerated Plants and in their Progeny" pages 1681-1689. see entire document
- CHEMICAL ABSTRACTS, Volume 98, No. 23, issued 1983, June 6 (Columbus, Ohio, USA), BECKER et al., "Herpes Simplex Virus Type 1 Thymidine Kinase Gene Expression in Escherichia Coli, "see page 168, column 2, the Abstract No. 192666q, Gene 1983, 21(1-2): 51-8 (Eng.).
- CHEMICAL ABSTRACTS, Volume 100, No. 9, issued 1984, February 27 (Columbus, Ohio, USA), KIT et al., "Nucleotide Sequence of the Herpes Simplex Virus Type 2 (HSV-2) Thymidine Kinase Gene and Predicted Amino Acid Sequence of Thymidine Kinase Polypeptide and its Comparison with the HSV-1 Thymidine Kinase Gene," see page 131, column 1, the Abstract No. 62615w, Biochim. Biophys. Acta 1983, 741(2): 158-70 (Eng.).
- CHEMICAL ABSTRACTS, Volume 100, No. 15, issued 1984, April 9 (Columbus, Ohio, USA), N. SH. ALIEVA, "Physiological and Biochemical Characteristics of Cotton Varieties Differing in their Resistance to Wilt," see page 327, column 1, the Abstract No. 117970r, K. Izuch. Rezist. Rast. Ekstremal'nykh Vozdeistv. Sredy 1982, 44-51 (Russ).
- CHEMICAL ABSTRACTS, Volume 103, No. 11, issued 1985, September 16 (Columbus, Ohio, USA), MACE et al., "Toxicity and Role of Terpenoid Phytoalexins in Verticillium Wilt Resistance in Cotton," see page 379, column 1, the Abstract No. 85240w, Physiol. Plant Pathol. 1985, 26(2): 209-218 (Eng.).
- CHEMICAL ABSTRACTS, Volume 100, No. 23, issued 1984, June 04 (Columbus, Ohio, USA), KHAMRAEV et al., "Potassium Fertilization: Yield and Quality," see page 499, column 2, the Abstract No. 190836f, Khlopkovodstvo 1984, 3: 24-5 (Russ.).
- CHEMICAL ABSTRACTS, Volume 105, No. 8, issued 1986, August 25 (Columbus, Ohio, USA), SARMINA et al., "Effect of Exposure to Energy on the Physicomechanical Properte of Cotton Fibers Treated with Trace Elements," see page 76, column 1, the Abstract No. 62035k, Izv. Akad. Nauk Tadzh. SSR, Otd. Fiz.-Mat., Khim. Geol. Nauk 1985, 4: 20-5 (Russ.).
- CHEMICAL ABSTRACTS, Volume 96, No. 15, issued 1982, April 12 (Columbus, Ohio, USA), RYAN et al., "The Metabolism of Chlortoluron, Diuron, and CGA 43 057 in Tolerant and Susceptible Plants," see page 236, column 1, the Abstract No. 117559n, Pestic. Biochem. Physiol. 1981, 16(3): 213-21 (Eng.).
- CHEMICAL ABSTRACTS, Volume 100, No. 11, issued 1984, March 12 (Columbus, Ohio, USA), KHUZHANAZAROV et al., "Effect of Copper and Zinc in Basic Fertilizer on the Productivity of Thin-Fibered Cotton," see page 468, column 1, the Abstract No. 84740k, Khim. Sel'sk Khoz. 1983, 12: 21-2 (Russ.).
- HANDBOOK OF PLANT CELL CULTURE, vol.2, no., 1984, page 487 - 510, PRICE,H.J. AND SMITH, R.H. 'CHAPTER 18: COTTON'

**Description**

BACKGROUND OF THE INVENTION

This invention is directed to plant regeneration and transformation of cotton, particularly cotton of the species Gossypium hirsutum L.

In recent years many tissues of diverse origin from plants belonging to different taxonomic groups have been established as in vitro tissue culture. Some of the factors controlling growth and differentiation of such cultures have also been determined. The establishment of subtle interactions among the different groups of plant hormones, and plant growth regulators operating either directly or indirectly, alone or in synergistic combination, have given to some degree an insight into certain interrelationships that may exist among cells, tissues and organs. The information is however by no means complete.

For some time it has been known that plant cell cultures can be maintained in a nondifferentiating proliferative state indefinitely. It has, however, only been recently found that redifferentiation of tissues, organs or whole plant organisms can be experimentally induced. Since the demonstrations by Skoog et al "Chemical regulation of growth and organ formation in plant tissues cultured in vitro," Symp. Soc. Exp. Biol. 11:18-130, 1958, incorporated herein by reference, that the relative ratio of a cytokinin to an auxin determines the nature of organogenesis in tobacco pith tissue. Reorganization or regeneration from callus cultures includes the formation of shoot primordia or embryos, both of which ultimately lead to plantlet development in vitro.

The tendency for organogenesis vs. embryogenesis still depends upon the species involved and the presence of certain triggering factors which are chemical and/or physical in nature.

In 1902, Haberlandt "Kulturversuche mit isolierten pflanzenzellen," Mat. Kl. Kais, Akad, Wiss. Wien 111:62, incorporated herein by reference, postulated that plant cells possessed the ability to produce entire plants and predicted that this would someday be demonstrable in cell cultures. In 1965, Reinert "Untersuchungen uber die morphogenese an Gewebekulturen," Ber. dt. Bot. Ges. 71:15, and Steward et al, "Growth and organized development of cultured cells/ II. Organization in cultures grown from freely suspended cells," Am. J. Bot. 45:705-708, working independently, confirmed the occurrence of in vitro somatic embryogenesis. Both references are incorporated herein by reference. In experimentally manipulating somatic embryogenesis it is believed that two components of the culture media, an auxin and the nitrogen source, play crucial roles.

It has also been shown that the process of somatic embryogenesis takes place in two stages: first, the induction of cells with with embryogenic competence in the presence of a high concentration of auxin; and second, the development of embryonic cell masses into embryos in the absence of or at a low concentration of auxin.

The induction of organogenesis or embryogenesis leads to distinct structural patterns in the callus. Detailed study of several plant species has enabled certain generalizations to be made about the developmental pathways leading to shoot, bud or embryo development.

The application of tissue culture techniques to the regeneration of plants via organogenesis or embryogenesis remains perhaps the most important contribution of basic studies in morphogenesis to commercial application.

Beasley reported the formation of callus in ovule cultures of cotton in 1971, "In vitro culture of fertilized cotton ovules," Bioscience 21:906:907, 1971, incorporated herein by reference. Later, Hsu et al "Callus induction by (2-chlorethyl) phosphonic (CPA) acid in cultured cotton ovules," Physiol. Plant 36:150-153, 1976, incorporated herein by reference, observed a stimulation of growth of calli obtained from ovules due to the addition of CPA and gibberellic acid to the medium. Callus cultures from other explants such as (a) leaf Davis et al., "In vitro culture of callus tissues and cell suspensions from okra (Hibiscus esculentus) and cotton (Gossypium hirsutum)," In vitro 9:395-398, 1974, both incorporated herein by reference; (b) hypocotyl Schenk et al. "Medium and technique for induction and growth of monocotyledonous and dicotyledonous plant cell cultures," Can. J. Bot. 50:199-204, 1972, incorporated herein by reference; and (c) cotyledons Rani et al. "Establishment of Tissue Cultures of Cotton," Plant Sci. Lett. 7:163-169, 1976, incorporated herein by reference) have been established for Gossypium hirsutum and G. arboreum.

Katterman et al, "The influence of a strong reducing agent upon initiation of callus from the germinating seedlings of Gossypium barbadense," Physiol. Plant 40:98-101, 1977, incorporated herein by reference, observed that the compact callus from cotyledons of G. barbadense formed roots, and in one instance regeneration of a complete plant was also obtained. Smith et al "Defined conditions for the initiation and growth of cotton callus in vitro, Gossypium arboreum," In vitro 13:329-334, 1977, incorporated herein by reference, determined conditions for initiation and subculture of hypocotyl-derived callus of G. arboreum. Subsequently, Price et al "Callus cultures of six species of cotton (Gossypium L) on defined media," Pl Sci. Lett. 8:115-119, 1977, and "Tissue culture of Gossypium species and its potential in cotton genetics and crop improvement," Beltwide Cotton Production Research Conference Proc. pp. 51-55, 1977, of the National Cotton Council, Memphis, each incorporated herein by reference, defined conditions for the initiation and subculture of callus from five species of Gossypium.

One of the common problems in establishing cultures of many plant species is the "browning" of the explant in the culture medium. In cotton, this leaching of polyphenols was overcome by replacing sucrose with glucose, and by transferring the cultures to a fresh medium every 10 days. After 3 or 4 passages on glucose supplemented medium, the browning completely disappeared and the cultures could be transferred back to sucrose-supplemented media. Although difficulties with the induction, browning and maintenance of calli during subcultures have been overcome with certain Gossypium species, all attempts to regenerate plants from callus cultures have been either unsuccessful or have involved several time-consuming steps. Davidonis and Hamilton "Plant Regeneration from Callus Tissue of Gossypium hirsutum," L. Plant Sci. Lett. 32:89-93, 1983, incorporated herein by reference, reported the eventual formation of embryos two years after the initiation of culture.

Although many growth substances, such as natural phytohormones and synthetic growth regulating compounds have been utilized in tissue culture media to bring about plant regeneration in vitro, no generalization, much less specifics, of the effects of different substances on plant regeneration has been arrived at. Indeed, the same substances, when applied to different plant species, may either inhibit growth, enhance growth, or have no effect whatsoever. Therefore, aside from certain standard procedures, it remains necessarily a difficult task to arrive at a working protocol for plant regeneration for any new species and by many orders of magnitude a more difficult task to achieve plant transformation.

Shoemaker, R.C. et al., Plant Cell Reports (1986), 178-181 relates to the characterisation of somatic embryogenesis and plant regeneration in cotton. It is further described that hypocotyl sections from cotton seed were cultured on media containing glucose (3%) as the carbohydrate source. Then, the developed calli were transferred to three different maintenance media. After 30 days on maintenance media, the calli were transferred to media containing glucose. After repeated transfers on said media, the call were placed onto media containing 3% sucrose. Finally, the major embryos were transferred to germination media.

Price, H. J. and Smith R. H., Cotton, Handbook of Plant Cell Culture (1984), Volume 2, 487-510 describes a method of somatic embryogenesis wherein hypocotyl segments of cotton seeds are cultured in a medium containing glucose as the carbon source. Suspension cultures of calli were grown in a medium containing sucrose as the carbon source. However, said document does not describe any intact plants recovered by this method.

EP-A-0 289 479 relates to a method for producing genetically transformed plants exhibiting toxicity to Coleopteran insects. Furthermore, transgenic plants are described which express the Coleopteran-type toxin protein of Bacillus thuringiensis at an insecticidal level.

The present invention provides a method for the rapid regeneration of cotton plants from segments excised from seedlings. The method described offers a high degree of repeatability and reliability and it enables genetic transformation of cotton plants.

Summary of the Invention

There is provided methods for the regeneration, with optional transformation, of a cotton plant from somatic cells.

Seed is sterilized and grown in the dark to a seedling. The seedling is one source of an explant, usually the hypocotyl and the cotyledon. Another source are immature zygotic embryos of developing fruit. The explant is subdivided and cultured in a first callus growth medium (containing glucose) for a period of time to allow a callus to develop from the explant on a culture medium which copes with phenolic secretions and stimulates cells of the explant to divide and proliferate. The callus, after passing through the phenolic secretion stage, is transferred to a fresh callus growth medium (containing sucrose) which develops the callus to an embryogenic callus. The embryo may then be subcultured to produce more embryogenic callus or transferred to another growth medium (plant germination medium) and cultured for a period of time sufficient to develop a plantlet which after another period of growth is transferred to a greenhouse, then into the field and grown to a mature plant from which seeds can be harvested.

The embryos may also be cultured in suspension. In this procedure, after the period of growth, the embryo containing embryogenic clumps greater than about 600 microns, preferably greater than about 800 microns in size are isolated and utilized for plant production. Smaller callus are recycled to the callus growth medium for growth to plant forming callus or maintained as an embryos source.

Transformation may occur at the explant, callus or suspension development stage. Transformation involves exposing the explant, callus and/or embryogenic callus to the action of an Agrobacterium vector containing an expressible gene sequence foreign to cotton for a time sufficient for the gene to transfer into the cells. The residual Agrobacterium is then killed off with an antibiotic which is toxic to the Agrobacterium. This is followed by selection of the transformed cells and/or embryogenic callus for development into transformed plantlets. In suspension culture, transformation and/or selection can occur prior to or following separation of embryogenic callus from cells and callus too immature to be embryogenic.

Plants of unique phenotypic traits are obtainable, and there is provided new cotton plants which having resistance to antibotics normally inhibitory to plant cell growth; cotton plants which have increased resistance or tolerance to herbicides, fungal pathogens and cotton plants which exhibit better yield and improved fiber quality.

Brief Description of the Drawings

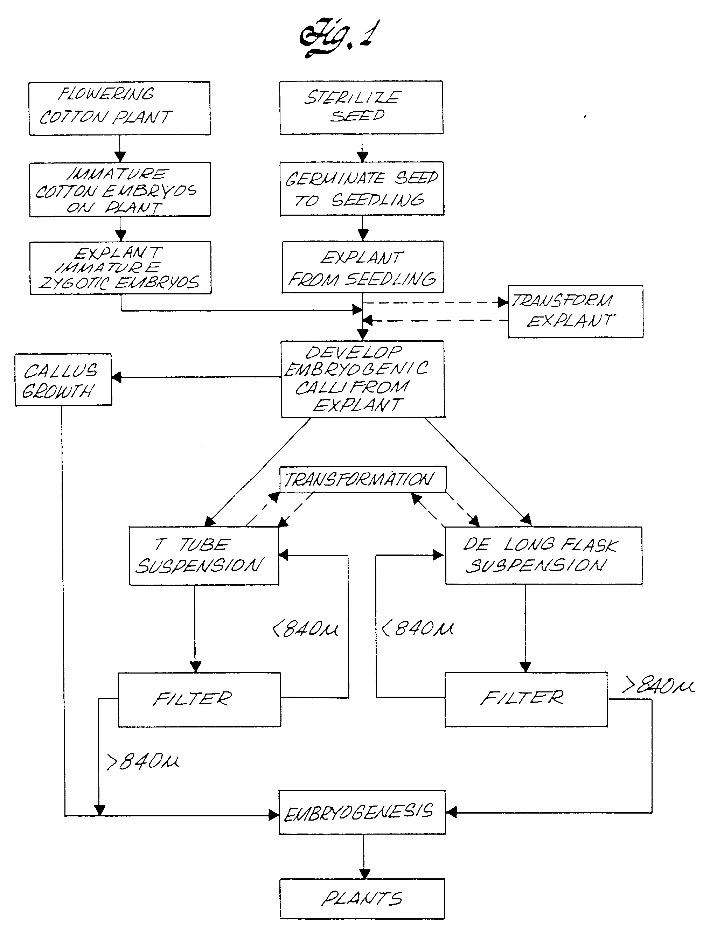

FIG. 1 presents diagramatically preferred procedures for development of cotton plants from seed by tissue culture techniques with a showing of establishing zones of transformation.

FIG. 2 is a photo illustration of embryogenic callus (10) of cotton with somatic embryos (12) at various stages of development including leaf (14) and root (16).

FIG. 3 is a photo illustration of a somatic cotton embryo at a late globular stage isolated to form the embryogenic callus culture as depicted in FIG. 2.

FIG. 4, as with reference to FIG. 2, is a photo illustration of embryos and young plantlets (18) of cotton developing on an embryo germination medium.

FIG. 5 is a photo illustration of small clumps of embryogenic cells from suspension cultures of cotton.

FIG. 6 is a photo illustration of a globular stage embryo from a suspension culture.

FIG. 7 illustrates germinating embryos obtained from suspension cultures showing emerging leaves (14) and roots (16).

FIG. 8 illustrates the development of plantlets of cotton growing on the embryo germination medium.

FIGS. 9 to 15 depict the genetic transformation of cotton, with FIG. 9 showing the development of cell colonies (20) from transformed cotton cells containing a gene for kanamycin resistance.

FIG. 10 shows somatic embryos developing from the selected antibiotic resistance cells of FIG. 9 on an antibiotic-supplemented medium.

FIG. 11 shows germinating embryos of transformed somatic embryos containing a gene confering resistance to the herbicide glyphosate.

FIG. 12 shows cotton plantlets developed from the embryos of FIG. 11.

FIG. 13 shows germinating somatic embryos transformed to confer resistance to Lepidopterous insects with leaf 14 and root 16 development.

FIG. 14 shows plantlets developed from the embryos of FIG. 13.

FIG. 15 shows a plantlet of the variety Siokra developed from transformed embryos exhibiting a resistance to kanamycin.

FIG. 16 shows the construction of mp 19/bt, a plasmid containing the 5' end of the Bt protoxin gene.

FIG. 17 shows the construction of mp 19/bt ca/del, a plasmid containing the CaMV gene VI promotor fused to the 5' end of Bt protoxin coding sequence.

FIG. 18 shows the construction of p702/bt, a plasmid having the 3' coding region of the protoxin fused to the CaMV transcription termination signals.

FIG. 19 shows the construction of pBR322/bt 14, containing the complete protoxin coding sequence flanked by CaMV promotor and terminator sequences.

FIG. 20 shows the construction of pRK252/Tn903/BglII.

FIG. 21 shows the construction of PCIB 5.

FIGS. 22 & 23 shows the construction of pCIB 4.

FIG. 24 shows the construction of pCIB 2.

FIG. 25 shows the construction of pCIB 10, a broad host range plasmid containing T-DNA borders and gene for plant selection.

FIG. 26 shows the construction of pCIB10/19Sbt.

FIG. 27 shows the construction of pCIB 710.

FIG. 28 shows the construction of pCIB10/710.

FIG. 29 shows the construction of pCIB10/35Sbt.

FIG. 30 shows the construction of pCIB10//35Sbt (KpnI).

FIG. 31 shows the construction of pCIB10/35Sbt (BcII).

FIG. 32 shows the construction of pCIB10/35Sbt (607)

FIG. 33 depicts the vector DEI PEP10.

FIG. 34 is a photo showing a field trial made up of cotton regenerants planted in a Verticillium infested field.

FIG. 35 is a photo showing progeny of a regenerated SJ4 plant in the field trial shown in FIG. 33. A somaclonal variant with improved tolerance to Verticillium fungus is indicated by the arrow.

Detailed Description

The present invention is directed to the regeneration by tissue culture of cotton plants particularly plants of the genus Gossyptium hirsutum from somatic cells for propagation in the field. Optionally, the cells may be transformed to include foreign genetic information.

The objects of the invention are solved by a method for the regeneration of a cotton plant from somatic cells which comprises the steps of:

a) providing a cotton explant;

b) culturing the explant in a first solid callus growth medium containing glucose for a period of time sufficient for phenolic secretion from the explant to enable undifferentiated callus to form the explant;

c) transferring the callus, after the phenolic secretion has stopped, to a second growth medium containing sucrose;

d) culturing the callus in the second callus growth medium for a period of time sufficient to allow development of embryogenic callus providing at least one embryo or embryogenic clumps;

e) transferring the embryo or embryogenic clumps to a plant germination medium; and

f) culturing the embryo or embryogenic clumps on the plant germination medium for a period of time sufficient to develop a plantlet from the embryo or embryogenic clumps with the proviso that cultivars Coker 201 and 315 are excluded.

In a preferred embodiment the explant in step a) is selected from the group consisting of hypocotyl, cotyledon and mixtures thereof derived from a cotton seedling and immature zygotic embryos.

In a still preferred embodiment the explant is developed by:

(i) sterilizing the seed in a first sterilizing solution;

(ii) rinsing the seed with sterile water;

(iii) sterilizing the seed in a second sterilizing solution;

(iv) rinsing the second sterilization medium from the seed with sterile water;

(v) transferring the seed to a seed germination medium; and

(vi) growing the seed in the seed germination medium in the dark for a period of time sufficient to produce a seedling; and

(vii) excising the explant from the seedling.

In a still preferred embodiment the first sterilizing solution is an aqueous solution containing 95% by volume ethanol and the second sterilizing solution is an aqueous solution containing 15% by weight sodium hypochlorite.

In a still preferred embodiment the seed germination medium in (v) of claim 3 is a basic agar medium and growth prior to explant removal is for a period of up to 14 days.

In a still preferred embodiment the first growth medium in step b) is a full or half-strength Murashige and Skoog growth medium.

In a still preferred embodiment the Murashige and Skoog growth medium contains naphthaleneacetic acid.

In a still preferred embodiment the Murashige and Skoog growth medium further contains thiamine hydrochloride, kinetin and inositol.

In a still preferred embodiment the Murashige and Skoog growth medium contains 0.4 mg/ml thiamine hydrochloride, 30g/l glucose, 2 mg/l naphthaleneacetic acid, 1 mg/l kinetin and 100 mg/l inositol.

In a still preferred embodiment the explant is cultured in step b) at a temperature of from 25 to 35°C under a daylight cycle of 16 hours of light and 8 hours of darkness.

In a still preferred embodiment the light intensity during the hours of light is 2000 to 4000 lux.

In a still preferred embodiment the explant is cultured in the first growth medium during a period of phenolic secretions from the developing callus with the medium changed at least every 10 days.

In a still preferred embodiment the explant is cultured for three to four weeks.

In a still preferred embodiment the second growth medium in step c) is a full or half-strength Murashige and Skoog growth medium.

In a still preferred embodiment the Murashige and Skoog growth medium contains naphthaleneacetic acid.

In a still preferred embodiment the Murashige and Skoog growth medium further contains a cytokinin.

In a still preferred embodiment the Murashige and Skoog growth medium contains from 1 to 10 mg/l naphthaleneacetic acid and from 0 to 1 mg/l cytokinin.

In a still preferred embodiment the callus is cultured in step d) at a temperature from 25 to 35°C under a daylight cycle of 16 hours of light and 8 hours of darkness.

In a still preferred embodiment the light intensity during the hours of light is 2000 to 4000 lux.

In a still preferred embodiment the callus is cultured for a period of time sufficient to form yellow to white embryogenic callus.

In a still preferred embodiment the embryogenic callus in step d) is subdivided and suspended in the second growth medium to form a suspension culture and said callus is grown to form embryogenic clumps of at least 600 $\mu$m in size.

In a still preferred embodiment the suspension culture at the beginning of growth contains from 750 to 1000 mg of callus parts per 8 ml second growth medium.

In a still preferred embodiment the embryogenic clumps of a size greater than 600 $\mu$m are filtered out.

In a still preferred embodiment the embryogenic clumps of a size less than 600 $\mu$m are resuspended in fresh second growth medium for further growth of embryogenic clumps.

In a still preferred embodiment embryogenic clumps greater than 800 $\mu$m are removed from the suspension for plantlet formation.

In a still preferred embodiment the plant germination medium in step e) contains a source of ammonium.

In a still preferred embodiment the plant germination medium in step e) is a Beasley and Ting's medium, rich in a source of nitrogen.

In a still preferred embodiment the Beasley and Ting's medium contains up to 500 mg/l casein hydrolysate and up to 1200 mg/l ammonium intrate.

In a still preferred embodiment the embryo or embroygenic clumps in step f) are cultured for a period of 2 to 3 weeks.

In a still preferred embodiment the plantlet in step f) is transferred to the soil under conditions of high humidity for a time sufficient to mature the plant to enable transfer to a hot house and then the field for growth to final maturity.

The various growth medium useful in accordance with this invention are as follows:

| SEED GERMINATION GROWTH MEDIUM | | |
| --- | --- | --- |
| COMPOSITION OF MODIFIED WHITE'S STOCK SOLUTION (Phytomorphology 11:109-127, 1961 incorporated herein by reference) | | |
| Component | Concentration per 1000 ml. | Comments |
| $MgSO_4.7 H_2O$ | 3.6 g | Dissolve and make up the final volume to 1000 ml. Label <u>White's A Stock</u>. Use 100 ml/l of final medium. |
| $Na_2SO_4$ | 2.0 g | |
| $NaH_2PO_4.H_2O$ | 1.65 g | |
| $Ca(NO_3)_2.4 H_2O$ | 2.6 g | Dissolve and make up the final volume to 1000 ml. Label <u>White's B Stock</u>. Use 100 ml/l of final medium. |
| $KNO_3$ | 800 mg | |
| KCl | 650 mg | |
| $Na_2MoO_4.2H_2O$ | 2.5 mg | Dissolve and make up the final volume to 100 ml. Label <u>White's C Stock.</u> Use 1.0 ml/l of final medium. |
| $CoCl_2.6H_2O$ | 2.5 mg | |
| $MnSO4.H_2O$ | 300 mg | |
| $ZnSO_4.7 H_2O$ | 50 mg | |
| $CuSO_4.5 H_2O$ | 2.5 mg | |
| $H_3BO_3$ | 50 mg | |
| Fe EDTA | | Use 10 ml/l of MSFe EDTA. |

(continued)

| SEED GERMINATION GROWTH MEDIUM | | |
|---|---|---|
| COMPOSITION OF MODIFIED WHITE'S STOCK SOLUTION (Phytomorphology 11:109-127, 1961 incorporated herein by reference) | | |
| Component | Concentration per 1000 ml. | Comments |
| Organic | | Use 10 ml/l of MS organic. |

| CALLUS GROWTH/MAINTENANCE MEDIUM | | |
|---|---|---|
| COMPOSITION OF MURASHIGE & SKOOG (MS) STOCK SOLUTIONS (Physiol. Plant 15:473-497, 1962 incorporated herein by reference) | | |
| Component | Concentration per 1000 ml. of Stock | Comments |
| $NH_4NO_3$ | 41.26 g | Dissolve and make up the final volume to 1000 ml. Use 40 ml/l of final medium. |
| $KNO_3$ | 47.50 g | |
| $CaCl_2.2 H_2O$ | 11.00 g | |
| $MgSO_4.7 H_2O$ | 9.25 g | |
| $KH_2PO_4$ | 4.25 g | |
| KI | 83 mg | Dissolve and make up the final volume to 1000 ml. Label <u>MS - Minor.</u> Use 10 ml/l of final medium. |
| $H_3BO_3$ | 620 mg | |
| $MnSO_4. H_2O$ | 1690 mg | |
| $ZnSO_4.7 H_2O$ | 860 mg | |
| $Na_2MoO_4.2 H_2O$ | 25 mg | |
| $CUSO_4.5 H_2O$ | 2.5 mg | |
| $CoCl_2.6 H_2O$ | 2.5 mg | |
| Nicotinic acid | 50 mg | Dissolve and make up the final volume to 1000 ml. Label <u>Ms - Organic.</u> Freeze in 10 ml aliquots. Use 10 ml/l of final medium. |
| Pyridoxin HCl | 50 mg | |
| Thiamine HCl | 10 mg | |
| Fe EDTA | 2.78 g | Dissolve 2.78 g of $FeSO_4.7 H_2O$ in about 200 ml of deionized water. Dissolve 3.73 g of $Na_2$ EDTA.2 $H_2O$ (disodium salt of ethylenediaminotetraacetic acid dihydrate) in 200 ml of deionized water in another beaker. Heat the $Na_2$ EDTA solution on a hot plate for about 10 minutes. While constantly stirring, add $FesO_4$ solution to $Na_2$ EDTA solution. Cool the solution to room temperature and make up the volume to 1000 ml. Label <u>MS EDTA.</u> Cover bottle with foil and store in refrigerator. Use 10 ml/l of final medium. |
| Fe $SO_4.7H_2O$ $Na_2$ EDTA.2 $H_2O$ | 3.73 g | |

(continued)

| CALLUS GROWTH/MAINTENANCE MEDIUM | | |
|---|---|---|
| COMPOSITION OF MURASHIGE & SKOOG (MS) STOCK SOLUTIONS (Physiol. Plant 15:473-497, 1962 incorporated herein by reference) | | |
| Component | Concentration per 1000 ml. of Stock | Comments |
| Thiamine HCl | 50 mg | Dissolve and make up the volume to 500 ml. Label <u>MS - Thiamine</u>. Use 4.0 ml/l of final medium. As if required. |
| Inositol | 10 g | Dissolve and make up |
| Glycine | 0.2 g | the final volume to 1000 ml. Label <u>Ms - glycine/inositol</u>. Use 10 ml/l of final medium. |

| PLANT GERMINATION MEDIUM | | |
|---|---|---|
| COMPOSITION OF BEASLEY AND TING'S STOCK SOLUTIONS (Am. J. Bot. 60:130-139, 1973 incorporated herein by reference) | | |
| Component | Concentration per 1000 ml. | Comments |
| $KH_2PO_4$ | 2.72 g | Dissolve and make up the volume to 100 ml. Label <u>B&T - A Stock</u>. Use 10 ml/l of final medium. |
| $H_3BO_3$ | 61.83 mg | |
| $Na_2MoO_4.2\,H_2O$ | 2.42 mg | |
| $CaCl_2.2\,H_2O$ | 4.41 g | Dissolve and make up the volume to 100 ml. Label <u>B&T - B Stock</u>. Use 10 ml/l of final medium. |
| KI | 8.3 mg | |
| $CoCl_2.6\,H_2O$ | 0.24 mg | |
| $MgSO_4.7\,H_2O$ | 4.93 g | Dissolve and make up the volume to 100 ml. Label <u>B&T - C Stock</u>. Use 10 ml/l of final medium. |
| $MnSO_4.H_2O$ | 169.02 mg | |
| $ZnSO_4.7\,H_2O$ | 86.27 mg | |
| $CUSO_4.5\,H_2O$ | 0.25 mg | |
| $KNO_3$ | 25.275 g | Dissolve and make up the volume to 200 ml. Label <u>B&T - D Stock</u>. Use 40 ml/l of final medium. |
| Nicotinic acid | 4.92 mg | Dissolve and make up the final volume to 100 ml. Label <u>B&T - Organics</u>. Use 10 ml/l of final medium. |
| Pyridoxin HCL | 8.22 mg | |
| Thiamine HCL | 13.49 mg | |
| Fe EDTA | | Use 10 ml/l of MS Fe EDTA. |
| Inositol | | 100 mg/l of final medium. |
| $NH_4NO_3$ (15 uM) | | 1200.6 mg/l of final medium. |

With any of the above solutions, the following procedure is used to prepare one liter of the medium. There is provided as a base, 200 ml of deionized water and the various stock solutions are added in the amounts stated for 1 liter. For example, if there is to be employed 10 ml of a stock in the final medium, then 10 ml of the stock are added to the 200 ml of the distilled water. To ensure the salts stay in solution, stock solutions are normally added in the order shown in the formulations above. After thoroughly mixing additional deionized water is added to the mixture to bring it to, as required 500 ml, and the mixture adjusted in pH to a value of from about 5.8 to 6.0. The final volume is brought to 1,000 ml and there is normally added tissue culture Agar, or its equivalent to a level of about 0.8% by weight. This is to provide some solidity to solution to reduce flow. The mixture is then autoclaved for about 5 to 20 minutes at a pressure 15-21 lbs/in$^2$ to kill any contaminating organism, and suitably labeled and stored as a sterile medium.

Briefly, cotton seeds are sterilized and germinated on a suitable seed germination medium such as a basal agar medium in the dark for a time sufficient to produce seedlings. The normal period of growth is up to about 4 weeks, typically 7 to 14 days.

Segments of explants are excised from the seedling. It is preferred that the explant come from the hypocotyl or cotyledon. In the alternative, one can equally use immature embryos obtained from the developing fruits of greenhouse or field grown cotton plants as the explant. The explant segments are cultured on a suitable first callus growth medium, preferably a or full Murashige and Skoog (MS) nutrient medium containing glucose. Growth occurs by culturing at a temperature of from about 25 to about 35°C in a light/dark cycle of about 16 hours of light and above 8 hours of dark. Culturing is the procedure whereby the medium is replaced at periodic intervals as the nutrients are consumed and continued for approximately about 3 to about 4 weeks, or until undifferentiated callus are formed. The callus are transferred to a second callus growth medium, preferably an MS medium supplemented with naphthaleneacetic acid (NAA) and sucrose as the carbon source and cultured for three to four months to produce embryos.

The embryos may then be maintained in the second callus growth medium to maintain an embryo supply or transferred to a plant germination medium such as Beasley and Ting's medium preferably containing casein hydrolysate and source of ammonuim cultured for 2 to 3 weeks to produce plantlets.

The plantlets are transferred to soil under high humidity conditions, then transplanted to larger pots in a greenhouse and finally transferred to the field for growth to maturity.

The methods briefly described herein have been successfully employed to induce somatic embryo formation in cotton of the species Gossypium hirsutum by tissue and suspension cultures and, ultimately, to obtain mature plants from hypocotyl and cotyledon derived callus cultures of Acala varieties of Gossypium hirsutum including SJ2, SJ4, SJ5, B1644, B1810, B2724, GC510 and C1 and non Acala "picker" Siokra and "stripper" variety FC 2017. Cultures have been transformed to normal plants with novel traits or properties.

More particularly, the procedure involves first the sterilizing of the cotton seeds. Suitable sterilization may be achieved by immersing the seeds in 95% ethanol for 2 to 3 minutes, rinsing in sterile water one or more times, then soaking the seeds in a 15% solution of sodium hypochlorite for 15 to 20 minutes, and rinsing several times with sterile water.

The sterilized seeds are then transferred to a first medium, termed a seed germination medium. A seed germination medium is one of normal salt content. A suitable germination medium is a basal agar medium, including White's medium or half-strength MS medium. (One-half ingredient strength). Germination normally occurs in the dark over an about 12 to about 14 day period.

Hypocotyl and/or cotyledons are preferably excised from the germinated seed, subdivided or cut into segments and cultured on a first callus growth medium such as an MS medium supplemented with growth substances. The presently preferred medium is the MS medium supplemented with about 0.4 mg/l thiamine hydrochloride, about 30 g/l glucose, about 2 mg/l naphthaleneacetic acid, about 1 mg/l kinetin, a common growth regulator, and about 100 mg/l inositol and agar. Thiamine hydrochloride can generally range in concentration from 0.1 to about 0.5 mg/l, glucose about 20 to about 30 g/l, about 1 to about 10 mg/l naphthaleneacetic acid, about 1 to about 2 mg/l kinetin and about 50 to about 100 mg/l inositol.

The cultures are maintained at a temperature of about 25 to about 35°C, preferably about 30°C and with a light/dark cycle of about 16 hours of light and about 8 hours of dark. It is preferred to have a light intensity of about 2000 to 4000 lux, more preferably about 3000 to 4000 lux.

The calli formed are periodically subcultured at 3 to 4 week intervals and transferred to a fresh first callus growth medium. In the culturing of the explants, secretions of phenolic compounds from the explants can occur as evidenced by darkening of the cultured medium. In this instance, the medium is changed more regularly. Darkening has been avoided by changing the culture medium every 10 days. Normally, after three to five medium changes, phenolic secretions will disappear. When this occurs, the first callus growth medium can be replaced by fresh callus growth medium containing sucrose or supplemented with sucrose as a carbon source.

After 3 to 4 weeks of culture, active calli develop on the cut surfaces of the explants. The calli are then transferred to a fresh second callus growth maintenance medium which is preferably an MS medium combined with about 1 to about 10 mg/l, preferably about 1 to about 5 mg/l NAA. Cytokinin is employed at a concentration of from 0 to about 1 g/l. A callus growth medium is characterized as a high salt content medium containing as much as 10 times more salt than the seed germination medium. The essential difference between first and second callus growth medium is the carbon source. Glucose is used during period of phenolic secretions. Sucrose is used when secretion have stopped.

The balance of the callus growth medium can remain the same or changed.

The calli are transferred in regular intervals to a fresh callus growth medium and, after generally about 5 to 7 passages or until an anthocyanin pigmentation becomes evident in a portion of the calli, which is followed by development of a yellowish-white embryogenic callus.

The embryogenic callus are then selectively subcultured and maintained by regular subculturing. The embryogenic callus contain somatic embryos at various stages of development. Some may have reached the point of development that enables growth into small plantlets. Most, however, require further development. Some may be advanced to germination. Other may be maintained as a source of embryos for future use.

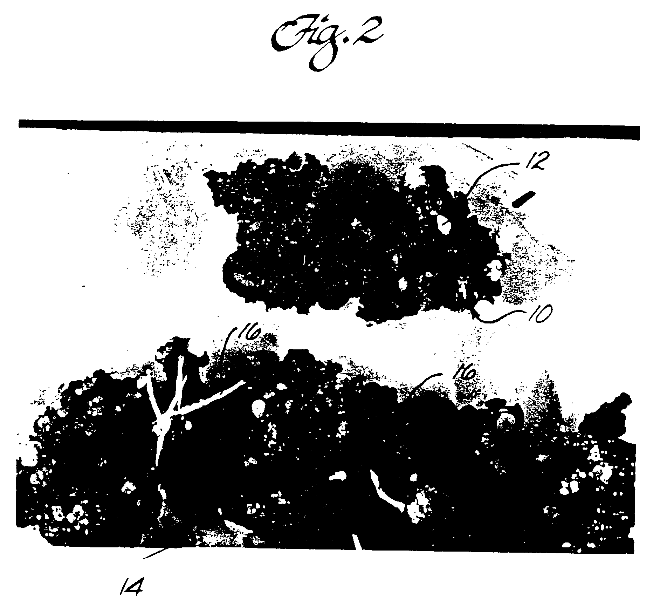

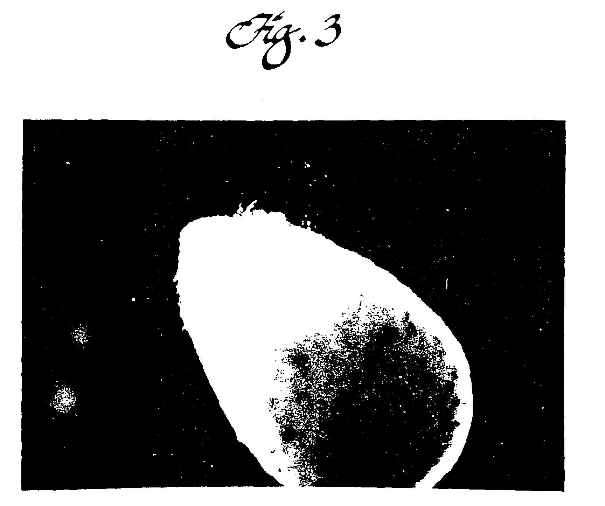

With reference to FIG. 2, there is illustrated this stage of development showing calli of Acala cotton 10 with somatic embryos 12 of differing size with some having emerging leaves 14 and roots 16. FIG. 3 illustrates a somatic embryo isolated at a late globular stage.

With reference to FIG. 4, further development may be achieved by transferring the somatic embryos to a third growth medium termed herein an embryo germination medium, a medium rich in nitrogen usually in the form of ammonia or its equivalent. Suitable media include Beasley and Ting's medium, preferably supplemented with up to about 500 mg/l casein hydrolysate.

Germination occurs from somatic embryos and, within 2 to 3 weeks, a well developed plantlet 18 of up to 6 leaves and good root system is generally formed.

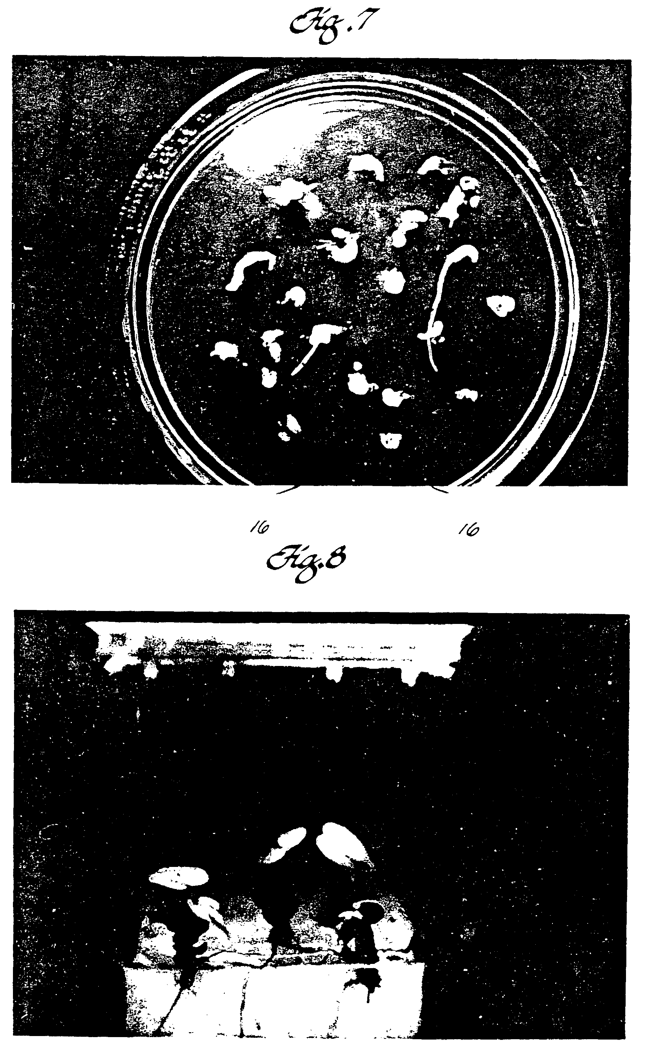

At this stage, the plantlets are transferred to soil in small clumps and grown in a standard incubator under conditions of high humidity. Temperature is normally maintained at about 25 to 30°C (See Fig. 7).

After a period of growth, the small plants are transferred to larger pots in a greenhouse and thereafter transferred to field and grown to maturity. All the regenerated plants are preferably self-pollinated either while growing in the green house or in field conditions and the seeds collected. Seeds are then germinated and 4 to 5 week old seedlings transferred to the field for progeny row trials and other standard plant breeding procedures. Practicing the above procedure produces viable cotton plants from about 35% of the explants in the period of time from about 6 to about 8 months.

Proliferation of Embryogenic Cotton Cells In Suspension Cultures

As an alternative to allowing the growing embryogenic calli to be developed into a plant, the callus may be cut into smaller pieces and further developed using suspension culture techniques.

In this procedure, suspension concentration is normally from about 750 to 1000 mg of callus parts to 8 ml. callus growth medium such as the second callus growth medium (MS medium supplemented with NAA), and allowed to grow in suspension. In a preferred embodiment, the suspension of the callus is inserted in T-tubes and placed on a roller drum rotating at about 1.5 rpm under a light regime of about 16 hours of light and about 8 hours of dark. Growth is for about 3 to 4 weeks.

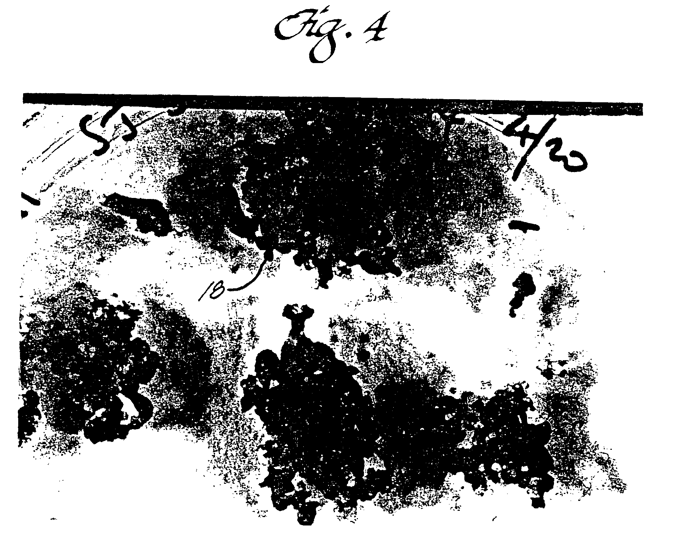

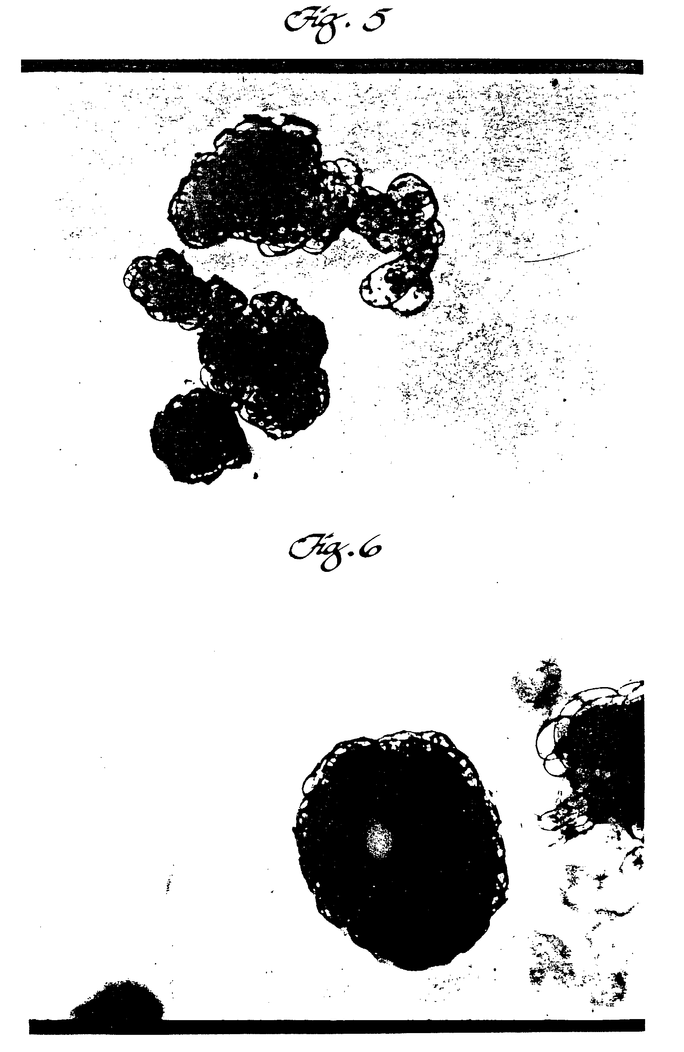

After about every 3 to 4 weeks, the suspension is filtered to remove large cell clumps of embryogenic callus depicted in groups in FIG. 5 and as isolated at late globular stages as shown in FIG. 6. The filtrate is returned to a nutrient medium for a 3 to 4 week period of growth. This procedure is repeated over and over with harvesting of large clumps at about 3 to 4 week intervals, at which time the medium is supplanted in whole or in part with fresh callus growth medium. Preferably, about 4 volumes or more of the fresh medium are added to about one volume of residual suspension. It is presently preferred that the filter employed have a mesh size greater than about 600 microns, preferably greater than 800 microns, as it has been observed the cell masses of a particle size less than 600 microns will not develop into plants, whereas cell masses greater than 600 microns and preferably greater than 800 microns have undergone sufficient differentiation so as to become embryogenic and capable of developing into viable plants.

Suspension cultures can also be initiated by transferring of embryogenic calli to a flask, such as a DeLong or Erlenmeyer flask, containing the liquid embryo growth medium in an amount of about 20 ml of MS and NAA at a concentration of 2.0 mg/l. The flask is placed on a gyrotory shaker and is shaken at about 100-110 strokes per minute. After 3 to 4 weeks the suspension is suitable for filtration as described above to remove the large cell clumps for plant development.

More typically, after the third or fourth subculture, the cell suspension from the "T" tube or De Long or Erlenmeyer flask is plated onto agar-solidified MS medium containing NAA (2.0 mg/l) or Beasley & Ting's medium containing casein hydrolysate (500 mg/l) media and a source of nitrogen. Within 3-4 weeks embryogenic calli with developing embryos become visible. Likewise, the larger cell clumps when plated on the above media give rise to embryogenic clumps with developing embryos.

In both suspension growth methods, the MS media is used to promote and/or sustain embryos whereas the germination medium is employed for rapid plant development.

The seedling explants, if desired, can be transformed. In this procedure, cotyledon and/or hypocotyl segments of the sterilized seed can be used. Cotyledons are preferred.

The segments are placed in a medium containing an Agrobacterium vector containing a code (genetic marker) such as resistance to an antibiotic, such as for instance kanamycin for a time sufficient for the vector to transfer the gene to the cells of the explant. Generally, contact times ranging from 1 minute to 24 hours may be used and may be accompanied with intermittent or gentle agitation. The explants are then removed and placed on agar-solidified callus growth medium such as a MS medium supplemented with NAA (2 mg/l) and incubated about 15 to 200 hours at 25 to 35°C, preferably 30°C, on a 16:8 hour light: dark regime.

After incubation, the explants are transferred to the same medium supplemented with the antibiotic cefotaxime preferably in a concentration of 200 mg/l. Cefotaxime is included to prevent any remaining Agrobacterium from proliferating and overgrowing the plant tissues. Alternatively, the explants can be rinsed with MS medium supplemented with NAA (2mg/l) and incubated an additional 4 to 28 days before rinsing, then incubating the same medium containing cefotaxime. At the end of 4-5 weeks of culture on fresh medium, the developing callus, i.e., primary callus, is separated from the remainder of the primary explant tissue and transferred to MS medium containing NAA (2 mg/l), cefotaxime (200 mg/l) and an antibiotic such as kanamycin sulfate (50 mg/l). Transformed primary callus, identified by virtue of its ability to grow in the presence of the antibiotic (kanamycin), is selected and embryos developed, germinated and plants obtained following the procedure set forth above.

It is also feasible to achieve transformation of a cell suspension. Following a normal subculture growth cycle of 7 to 14 days, usually 7 to 10 days, cells are allowed to settle leaving a supernatant which is removed. The remaining concentrated suspended cells may be centrifuged at 4000Xg for 5 minutes and the excess medium is discarded. The concentrated suspension cultures are resuspended in the 8 ml of the same medium which contains the Agrobacterium. The suspension is transferred to "T" tubes and suitably agitated for incubation.

Following about 2 to 24 hours, preferably 3 to 5 hours, of incubation to allow for bacterial attachment and DNA transfer, the suspension is removed and allowed to settle. The supernatant containing the bacteria is discarded and the cells are washed with fresh medium. The suspension may, if desired, be centrifuged for about 5 minutes and the supernatant removed. In either event, the cells are resuspended in the same medium and transferred to a "T" tube or flask and suspension subculture resumed. The object is to minimize the amount of unattached Agrobacterium vector left in the cell suspension.

After about 15 to about 200 hours, typically 15 to about 72 hours, preferably 18 to 20 hours, the suspension is filtered to remove large clumps and washed with fresh liquid medium and allowed to settle. The suspension is resuspended in the fresh liquid medium containing cefotaxime (200 mg/l) plated on a solidified medium in Petri dishes.

Alternatively, the suspension may be resuspended in fresh medium containing cefotaxime and allowed to grow an additional 4 to 28 days prior plating on solidified medium in Petri dishes. Cell concentration is 1 vol. of suspension cells plus 3 vol. of medium with cefotaxime. Kanamycin at 10 to 300 mg/l preferably about 20 to 200 mg/l more preferably about 40 to 80 mg/l is included in the medium for selection of transformed cells expressing the neomycin phosphotransferase (NPT) gene. Cells and embryos proliferating in the selective concentration of kanamycin are further grown as set forth above to mature somatic embryos capable of germinating and regenerating into whole plants according to the procedures described herein.

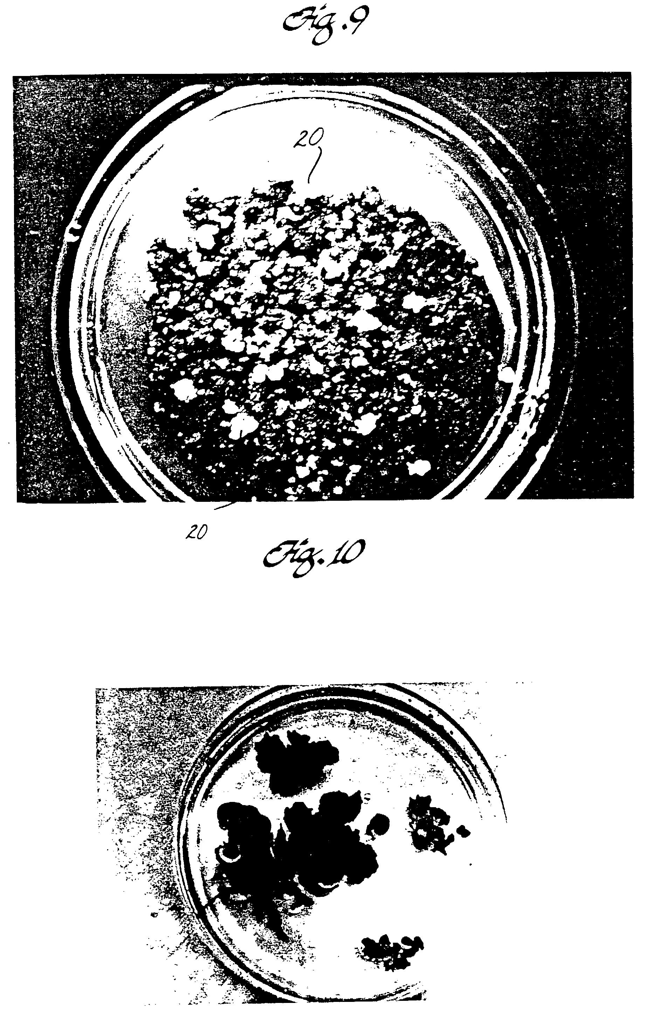

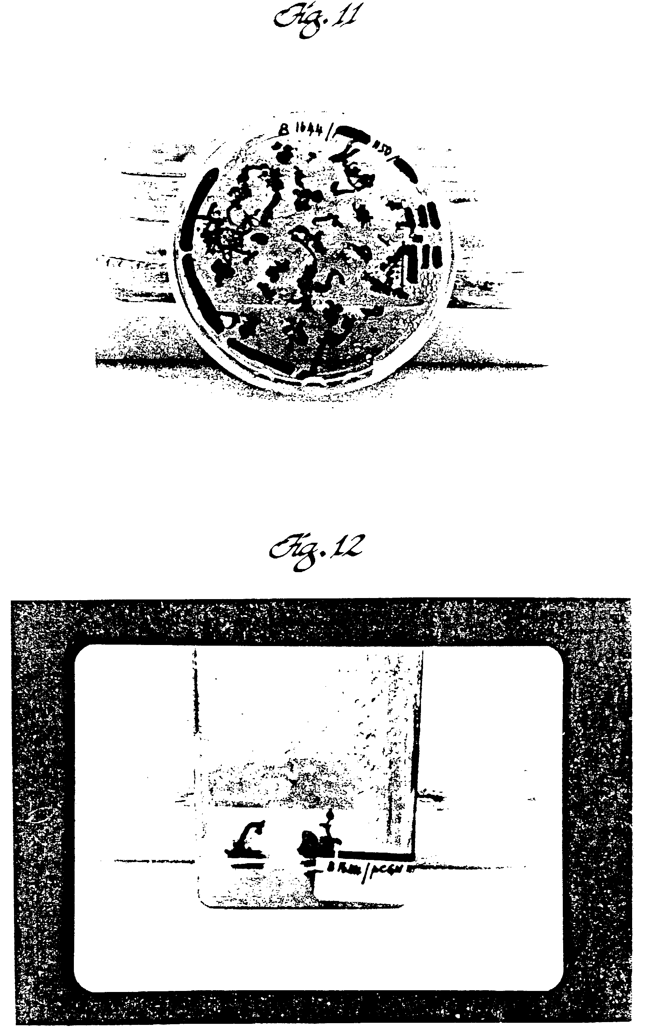

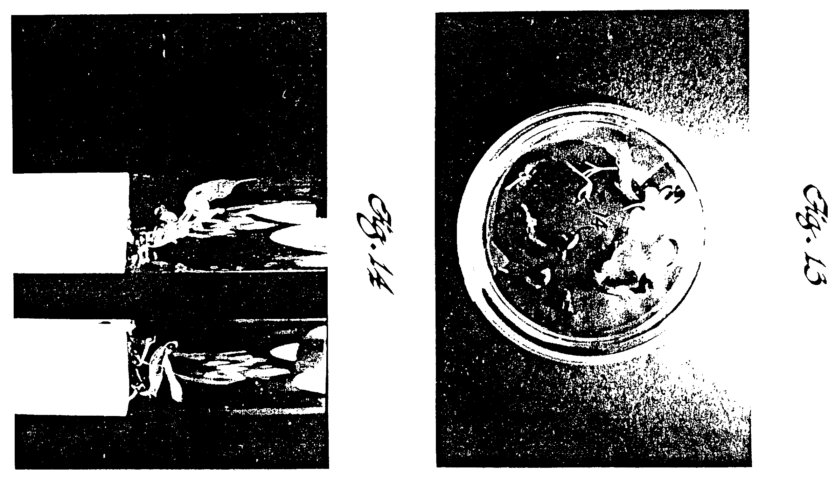

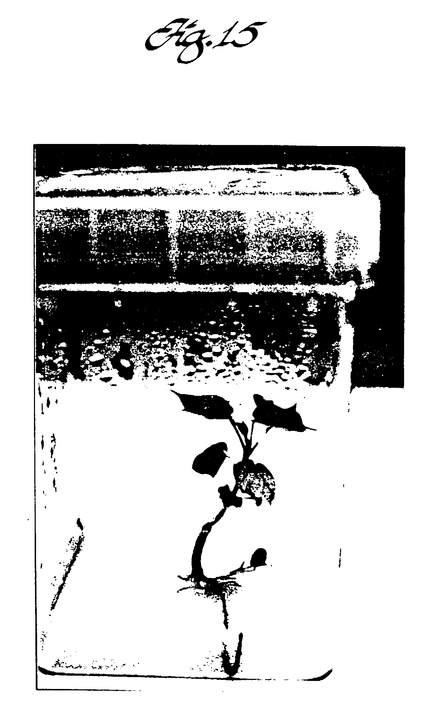

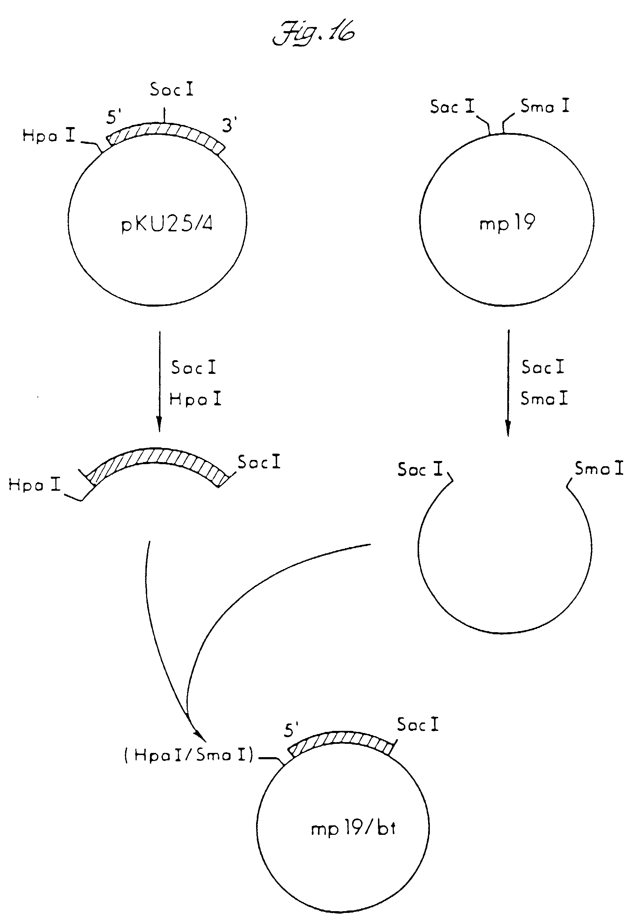

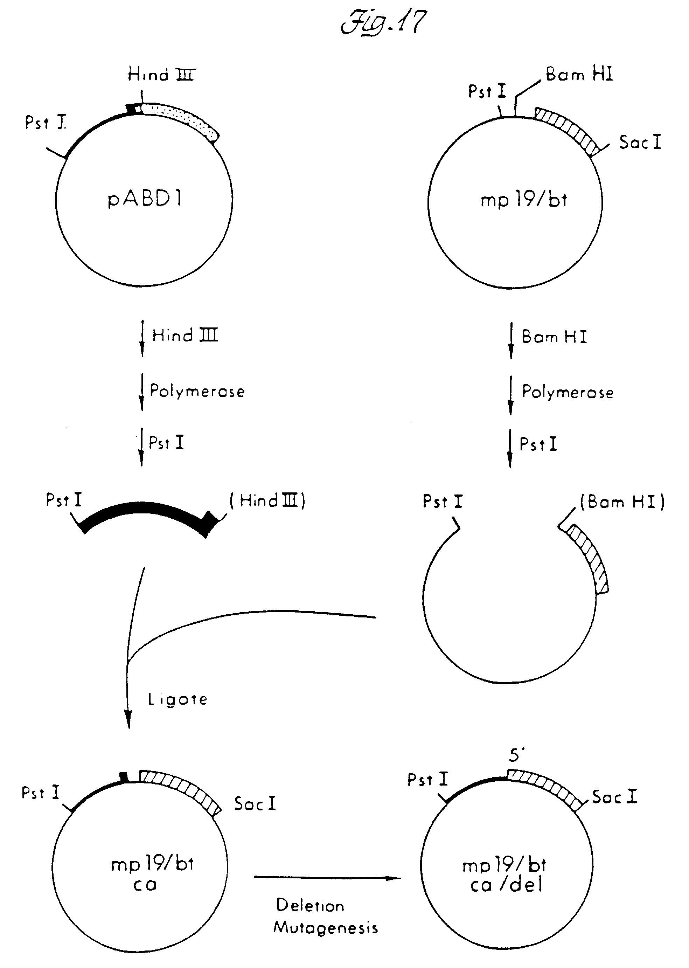

Using the above procedure and with reference to FIG. 9, there is shown variable cell colonies which is consequence of transformation. There exists cotton cells 20 exhibiting resistance to the antibiotic kanamycin. With reference to FIG. 10, transformed calli are shown developing into somatic embryos on an antibiotic MS medium. FIG. 11 shows transformed somatic embryos established to have kanamycin resistance and transformed to have resistance to the herbicide glyphosate. FIG. 12 shows plants from the embryos of FIG. 11. FIG. 13 shows cells transformed to have resistance to lepidopterous insects growing on an MS medium and in FIG. 14 transferred to a Beasley and Ting's medium whereas FIG. 15 shows further development of the plantlets of FIG. 14 to more mature plantlets.

COTTON REGENERATION

Example 1

Regeneration of plants starting from cotyledon explants

Seeds of Acala cotton variety SJ2 of Gossypium hirsutum were sterilized by contact with 95% alcohol for three minutes, then twice rinsed with sterile water and immersed with a 15% solution of sodium hypochlorite for 15 minutes, then rinsed in sterile water. Sterilized seeds were germinated on a basal agar medium in the dark for approximately 14 days to produce a seedling. The cotyledons of the seedlings were cut into segments of 2-4mm$^2$ which were transferred aseptically to a callus inducing medium consisting of Murashige and Skoog (MS) major and minor salts supplemented with 0.4 mg/l thiamine-HCl, 30 g/l glucose, 2.0 mg/l naphtaleneacetic acid (NAA), 1 mg/l kinetin, 100 mg/l of m-inositol, and agar (0.8%). The cultures were incubated at about 30°C under conditions of 16 hours light and 8 hours darkness in a Percival incubator with fluorescent lights (cool daylight) providing a light intensity of about 2000-4000 lux.

Calli were formed on the cultured tissue segments within 3 to 4 weeks and were white to gray-greenish in color. The calli formed were subcultured every three to four weeks onto a callus growth medium comprising MS medium containing 100 mg/l m-inositol, 20 g/l sucrose, 2 mg/l naphthaleneacetic acid (NAA) and agar. Somatic embryos formed four to six months after first placing tissue explants on a callus inducing medium. The callus and embryos were maintained on a callus growth medium by subculturing onto fresh callus growth medium every three to four weeks.

Somatic embryos which formed on tissue pieces were explanted either to fresh callus growth medium, or to Beasley & Ting's medium (embryo germination medium).

The somatic plantlets which were formed from somatic embryos were transferred onto Beasley and Ting's medium which contained 1200 mg/l ammonium nitrate and 500 mg/l casein hydrolysate as an organic nitrogen source. The medium was solidified by a solidifying agent (Gelrite) and plantlets were placed in Magenta boxes.

The somatic embryos developed into plantlets within about three months. The plantlets were rooted with six to eight leaves and about three to four inches tall and were transferred to soil and maintained in an incubator under high humidity for three to four weeks and then transferred to a greenhouse. After hardening, plants were also transferred to open tilled soil.

Example 2

The procedure of Example 1 was repeated using instead half-strength MS medium in which all medium components have been reduced to one-half the specified concentration. Essentially the same results were obtained.

Example 3

The procedures of Examples 1 and 2 were repeated except that the explant was the hypocotyl segments. The same results were obtained.

Example 4

The procedure of Examples 1 and 2 were repeated except that the explant was the immature zygotic embryo. Essentially the same results were obtained.

Example 5

The procedure of Examples 1 and 2 was repeated with Acala cotton varieties SJ4, SJ5, SJ2C-1, GC510, B1644, B2724, B1810, the picker variety Siokra and the stripper variety FC2017. All were successfully regenerated.

Example 6

The procedure of Example 1 was repeated to the extent of obtaining callus capable of forming somatic embryos. Pieces of about 750-1000 mg of actively growing embryogenic callus was suspended in 8 ml units of liquid suspension culture medium comprised of MS major and minor salts, supplemented with 0.4 mg/l thiamine HCl, 20 g/l sucrose, 100 mg/l of inositol and naphthaleneacetic acid (2 mg/l) in T-tubes and placed on a roller drum rotating at 1.5 rpm under 16:8 light: dark regime. Light intensity of about 2000-4500 lux was again provided by fluorescent lights (cool daylight).

After four weeks, the suspension was filtered through an 840 micron size nylon mesh to remove larger cell clumps. The fraction smaller than 840 microns were allowed to settle, washed once with about 20-25 ml of fresh suspension culture medium. This suspension was transferred to T-tubes (2 ml per tube) and each tube diluted with 6 ml of fresh suspension culture medium. The cultures were maintained by repeating the above procedure at 10-12 day intervals. Namely, the suspension was filtered and only the fraction containing cell aggregates smaller than 840 microns was transferred to fresh suspension culture medium. In all instances, the fraction containing cell clumps larger than 840 microns was placed onto the callus growth medium to obtain mature somatic embryos.

The somatic embryos that were formed on callus growth medium were removed and transferred to embryo germination medium and using the protocol of Example 1 were germinated, developed into plantlets and then field grown plants.

Example 7

The procedure of Example 6 was repeated except that suspension cultures were formed by transferring 750-1000 mg of embryogenic calli to a DeLong flask containing 15-20 ml of the MS liquid medium containing 2 mg/l NAA. The culture containing flask was placed on a gyrotory shaker and shaken at 100-110 strokes/minute. After three weeks the suspension was filtered through an 840 micron nylon mesh to remove the large cell clumps for plant growth, as in Example 4. The less than 840 micron suspension was allowed to settle, washed once in the MS liquid medium and resuspended in 2 to 5 ml of the MS liquid medium. The suspension was subcultured by transfer to fresh medium in a DeLong flask containing 1-2 ml of suspension and 15 ml of fresh MS liquid medium. The cultures are maintained by repeating this procedure at seven to ten day intervals. At each subculture only the less than 840 micron' suspension was subcultured and the large clumps (840 microns or greater) were used for plant growth.

Example 8

After three or four subcultures using the suspension growth procedure of Examples 6 and 7, 1.5 to 2.0 ml of cell suspension from the T-tube and DeLong flask were in each instance plated onto agar-solidified MS medium containing 2 mg/l NAA and Beasley & Ting medium containing 500 mg/l casein hydrolysate. Within three to four weeks embryogenic calli with developing embryos became visible. Again, the 840 micron or greater cell clumps were plated on the callus growth medium giving rise to embryogenic clumps with developing embryos which ultimately grew into plants.

COTTON TRANSFORMATION

Example 9

Transformation To Form Tumorous-Phenotype With Agrobacteria LBA 4434

An Acala cotton suspension culture was subcultured for three to four months in T-tubes with the medium (MS medium containing 2 mg/l NAA) being changed every seven to ten days. After any medium change thereafter the cells can be allowed to settle and harvested for transformation. The supernatant was removed by pipeting and cells transformed with the Agrobacterium strain LBA 4434. The Agrobacterium strain LBA 4434 is described in (Hoekema, A. et al. Nature 303: 179-180, 1983, incorporated herein by reference) contains a Ti plasmid-derived binary plant transformation system. In such binary systems, one plasmid contains the T-DNA of a Ti-plasmid, the second plasmid contains the vir-region of a Ti-plasmid. The two plasmids cooperate to effect plant transformation. In the strain LBA 4434, the T-DNA plasmid, pAL 1050, contains $T_L$ of pTiAch5, an octopine Ti-plasmid and the vir-plasmid in strain LBA 4434, pAL4404, contains the intact virulence regions of pTiAch 5 (Ooms, G. et al. Plasmid 7:15-29, 1982, incorporated herein by reference). Strain LBA 4434 is available from Dr. Robert Schilperoort of the Department of Biochemistry, University of Leiden, The Netherlands.

The transforming Agrobacterium strain was taken from a glycerol stock, inoculated in a small overnight culture, from which a 50-ml culture was inoculated the following day. Agrobacteria was grown on YEB medium containing per liter in water adjusted to pH 7.2 with NaOH, 5 g beef extract, 1 g yeast extract, 5 g peptone, 5 g sucrose. After autoclaving, 1 ml of 2 M $MgCl_2$ is added after which antibiotics, as required to kill other strains. The absorbance at 600 nm of the 50 ml overnight culture is read, the culture centrifuged and the formed pellet resuspended in the plant cell growth medium (MS medium plus NAA at 2 mg/l) to a final absorbance at 600 nm of 0.5.

Eight ml of this bacterial suspension of Agrobacterium LBA 4434 was added to each T-tube containing the suspension plant cells after removal of the supernatant liquid. The T-tube containing the plant and bacteria cells was agitated to resuspend the cells and returned to a roller drum for three hours to allow the Agrobacteria to attach to the plant cells. The cells were then allowed to settle and the residual supernatant removed. A fresh aliquot of growth medium was added to the T-tube and the suspension allowed to incubate on a roller drum for a period of 18 to 20 hours in the presence of any residual Agrobacteria which remained. After this time, the cells were again allowed to settle, the supernatant removed and the cells washed twice with a solution of growth medium containing cefotaxime (200 ug/ml). After washing, the cells from each T-tube were resuspended in 10 ml growth medium containing cefotaxime (200 ug/ml in all cases) and 1 ml aliquots of the suspension plated on petri dishes.

Infected cells grew on the growth medium to which no phytohormones were added establishing the tissue had received the wild-type phytohormone genes in T-DNA. The cells developed tumors, further indicating transformation of the cultures.

Example 10

Transformation of Cotton To Form a Kanamycin-Resistant Non-Tumorous Phenotype

The suspension culture as obtained in Example 9 was transformed using an Agrobacteria which contained the T-DNA containing binary vector pCIB 10. (Rothstein, S.J. et al. Gene 53: 153-161, 1987, incorporated herein by reference) as well as the pAL 4404 vir-plasmid. The T-DNA of pCIB 10 contains a chimeric gene composed of the promoter from nopaline synthase, the coding region from Tn5 encoding the enzyme neomycin phosphotransferase, and the terminator from nopaline synthase. The Agrobacteria containing pCIB 10 were grown on YEB medium containing kanamycin (50 ug/ml). Transformation was accomplished in the same manner as in Example 10 except that the 1 ml aliquots resulting in cells and Agrobacteria were immediately plated on selective media containing either kanamycin (50 ug/ml) or G418 (25 ug/ml). Expression of the nos/neo/nos chimeric gene in transformed plant tissue allows the selection of this tissue in the presence of both antibiotics. The existence in two to four weeks of transformed tissue became apparent on the selection plates. Uninfected tissue as well as added control tissue showed no signs of growth, turned brown and died. Transformed tissue grew very well in the presence of both kanamycin and G418.

At this time, tissue pieces which were growing well were subcultured to fresh selection medium. Somatic embryos formed on these tissue pieces and were explanted to fresh non-selective growth media. When the embryos began to differentiate and germinate, i.e., at the point where they were beginning to form roots and had two or three leaves, they were transferred to Magenta boxes containing growth medium described in Example 1. Growth was allowed to proceed until a plantlet had six to eight leaves, at which time it was removed from the agar medium.

The plantlets were now placed in potting soil, covered with a beaker to maintain humidity and placed in a Percival incubator for four to eight weeks. At this time, the plant was removed from the beaker and transferred to a greenhouse. The plants grew in the greenhouse, flowered and set seed.

Example 11

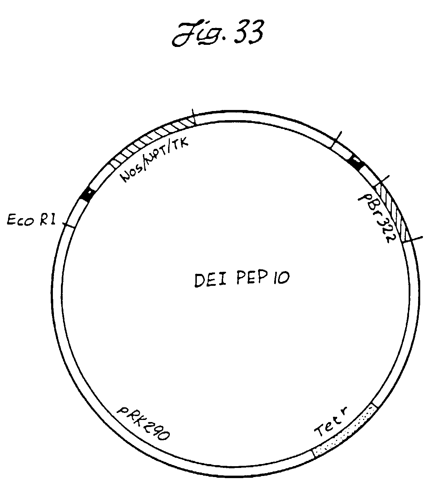

The procedure of Example 10 was followed, except that the transforming Agrobacteria used contained the T-DNA vector DEI PEP10 as well as the pAL4404 vir plasmid. DEI PEP10, shown in Figure 33, utilizes two T-DNA PstI cleaved right border sequences from A. Tumefaciens (strain C-58) which had been further subdivided with BamHI for integration in the plant genome, a passenger maize phosphoenolpyruvate carboxylase gene (Pepcase gene), and a chimeric gene (NOS/NPT/TK) capable of expression in plants and conferring resistance to the antibiotics kanamycin and G418. This chimeric gene utilizes a nopaline synthetase promoter, the neomycin phosphotransferase II coding region from Tn5, and the terminator from the herpes simplex virus thimidine kinase gene. Following transformation, embryogenic callus and embryos were obtained by selection on kanamycin (50 mg/l). No resistant callus was obtained from the control (non-transformed callus) plated on kanamycin at this level (50 mg/l).

Example 12

Transformation of Cotton Suspension Culture Cells To A Glyphosate-Tolerant Phenotype

The procedure of Example 10 was followed, except that the transforming Agrobacteria used contained the T-DNA vector pPMG85/587 (Fillatti, J. et al., Mol Gen. Genet. 206: 192-199, 1987, incorporated herein by reference) as well as the pAL4404 vir plasmid. The plasmid pPMG85/587 carries three chimeric genes capable of expression in plants. Two genes code for neomycin phosphotransferase (NPT) which confers resistance to the antibiotics kanamycin and G418. The third chimeric gene, containing the coding sequence from a mutant aroA gene of S. typhimurium, confers tolerance to the herbicide glyphosate (Comai, et al., Science 221: 370-371, 1983, incorporated herein by reference). The Agrobacteria containing pPMG85/587 were grown on medium containing kanamycin (100 ug/ml). Transformation is accomplished as detailed in Example 10 except that the suspension is allowed to grow for 28 days at which time 1 ml aliquots were plated on medium containing selective antibiotics. Expression of the NPT chimeric gene in transformed plant tissue allowed selection of this tissue on both antibiotics. In this instance the selective antibiotic was kanamycin (50 ug/ml).

In two to four weeks, transformed tissue became apparent on the selection plates. Plant tissue, individual embryos and callus were then placed on growth medium containing the herbicide glyphosate 1mM and transformed tissue continued to grow well. Extraction and analysis of the proteins of both callus and embryos confirmed the presence of the product of the glyphosate tolerance gene.

## Example 13

### Transformation of Cotton Suspension Culture Cells To a Hygromycin-Resistant Non-Tumorous Phenotype

The transformation procedure of Example 10 was followed except there was used as the transforming Agrobacteria one containing the T-DNA binary vector pCIB 715 (Rothstein, S. J. et al. Gene 53: 153-161, 1987) as well as the vir plasmid. The T-DNA of pCIB 715 contains a chimeric gene composed of the promoter and terminator from the cauliflower mosaic virus (CaMV) 35S transcript (Odell et al, Nature 313: 810-812, 1985, incorporated herein by reference) and the coding sequence for hygromycin B phosphotransferase (Gritz, L. and J. Davies, Gene 25: 179-188, incorporated herein by reference). Agrobacteria containing pCIB 715 was grown on YEB containing kanamycin (50 ug/ml).

Transformation was accomplished as detailed in Example 10 again with the change that the 1 ml aliquots were plated immediately on medium containing as the selective antibiotic 50 ug/ml hygromycin. Expression of the chimeric hygromycin gene in transformed plant tissue allows the selection of this tissue on the medium containing hygromycin. Transformed tissue was grown in the manner described in Example 8 on the selection growth medium establishing transformation had occurred.

## Example 14

### Transformation of Cotton Suspension Culture Cells To Confer Resistance To Lepidopteran Insects

The procedure of Example 10 was followed except where changes are noted below. Different transforming Agrobacteria were used. Also, after plant tissue was selected on an antibiotic for the selection of transformed material, it was further selected for expression of the BT gene as defined herein.

The Agrobacteria used contained the T-DNA vector pCIB10 (Rothstein et al, Gene 53:153-161 (198) incorporated herein by reference into which had been inserted the following chimeric Bacillus thuringiensis endotoxin genes ("BT Genes"):

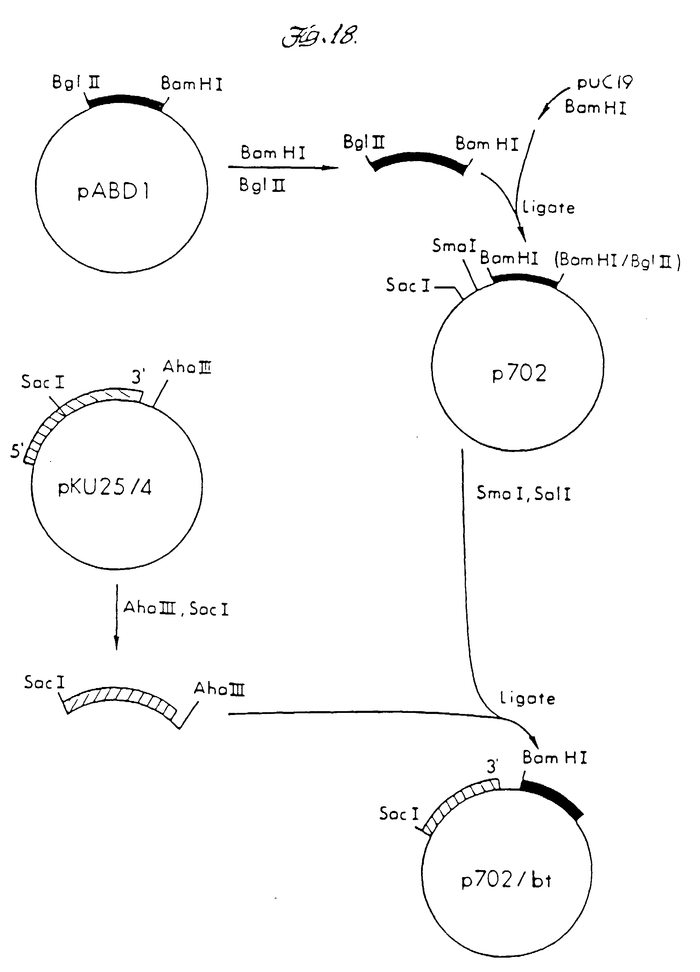

To prepare the Agrobacterium vector there was fused the CaMV gene VI promotor and protoxin coding sequences. A derivative of phage vector mp19 (Yanish-Perron et al., 1985) was first constructed. The steps are shown in FIGS. 16 and 17. First, a DNA fragment containing approximately 155 nucelotides 5' to the protoxin coding region and the adjacent approximately 1346 nucleotides of coding sequence are inserted into mp19. Phage mp19 ds rf (double-stranded replicative form) DNA was digested with restriction endonucleases SacI and SmaI and the approximately 7.2-kbp vector fragment was purified after electrophoresis through low-gelling temperature agarose by standard procedures. Plasmid pKU25/4, containing approximately 10 kbP (kilobase pairs) of Bacillus thuringiensis DNA, including the protoxin gene, was obtained from Dr. J. Nueesch, CIBA-Geigy Ltd., Basle, Switzerland. The nucleotide sequence of the protoxin gene present in plasmid pkU25/4 is shown in Formula 1 below. Plasmid pKU25/4 DNA was digested with endonucleases HpaI and SacI, and a 1503 bp fragment containing nucleotides 2 to 1505 of Formula 1 and purified. This fragment contains approximately 155 bp of bacteria promotor sequences and approximately 1346 bp of the start of the protoxin coding sequence. Approximately 100 ng of each fragment is then mixed, T4 DNA ligase added, and incubated at 15°C overnight. The resulting mixture was transformed into E. coli strain HB 101, mixed with indicator bacteria E. coli JM 101 and plated. One phage (mp19/bt) was used for further construction below.

Next, a fragment of DNA containing the CaMV gene VI promotor, and some of the coding sequences for gene VI, was inserted into mp19/bt. Phage mp19/bt ds rf DNA is digested with BamHI, treated with the large fragment of DNA polymerase to create flush ends and recleaved with endocuclease PstI. The larger vector fragment was purified by electrophoresis as described above. Plasmid pABD1 is described in Paszkowski et al., EMBO J. 3, 2717-2722, (1984) incporated herein by reference. Plasmid pABD1 DNA is digested with PstI and HindIII. The fragment approximately 465 bp long containing the CaMV gene VI promotor and approximately 75 bp of gene VI coding sequence was purified. The two fragments were ligated and plated as described above. One of the resulting recombinant phages, mp19/btca contained the CaMV gene VI promotor sequences, a portion of the gene VI coding sequence, approximately 155 bp of Bacillus thuringiensis DNA upstream of the protoxin coding sequence, and approximately 1346 bp of the protoxin coding sequence. To fuse the CaMV promotor sequences precisely to the protoxin coding sequenes, the intervening DNA was deleted using oligionicleotide-directed mutagensis of mp19/btca DNA. A DNA oligonucleotide with the sequence (5') TTCGGATTGTTATCCATGGTTGGAGGTCTGA (3) was synthesized by routine procedures using an Applied Biosystems DNA Synthesizer. This oligonucleotide is complimentary to those sequences in phage mp19/btca DNA at the 3' end of the CaMV promoter (nucleotides 5762 to 5778 in Hohn, Current Topics, in Microbiology and Immunology, 96, 193-235 (1982) incorporate herein by reference and the beginning of the protoxin coding sequence (nucleotides 156 to 172 in formula I above). The general procedure for the mutagensis is that described in Zoller and Smith, Meth, Enzym., 100 468-500 (1983) incorporated herein by reference. Approximately five micrograms of single-standed phage mp19/btca DNA was mixed with 0.3 mg of phosphorylated oligonucleotide in a volume of 40 ul. The mixture was heated to 65°C for 5 min, cooled to 50°C, and slowly cooled to 4°C. Next, buffer, nucleotide triphosphates, ATP, $T_4$ DNA ligase and large fragment of DNA polymerase were added and incubated overnight at 15°C as described [Zoller and Smith Meth. Enzym., 100, 468-500 (1983)] incorporated herein by reference. After agarose gel electrophoresis, circular double-stranded DNA was purified and transfected into E. coli strain JM101. The resulting plaques are screened for sequences that hybridize with 32P-labeled oligonucleotide, and phage are analyzed by DNA restriction endonuclease analysis. Among the resulting phage clones were ones which have correctly deleted the unwanted sequences between the CaMV gene VI promotor and the protoxin coding sequence. This phage is called mp19/btca/del (see FIG. 17).

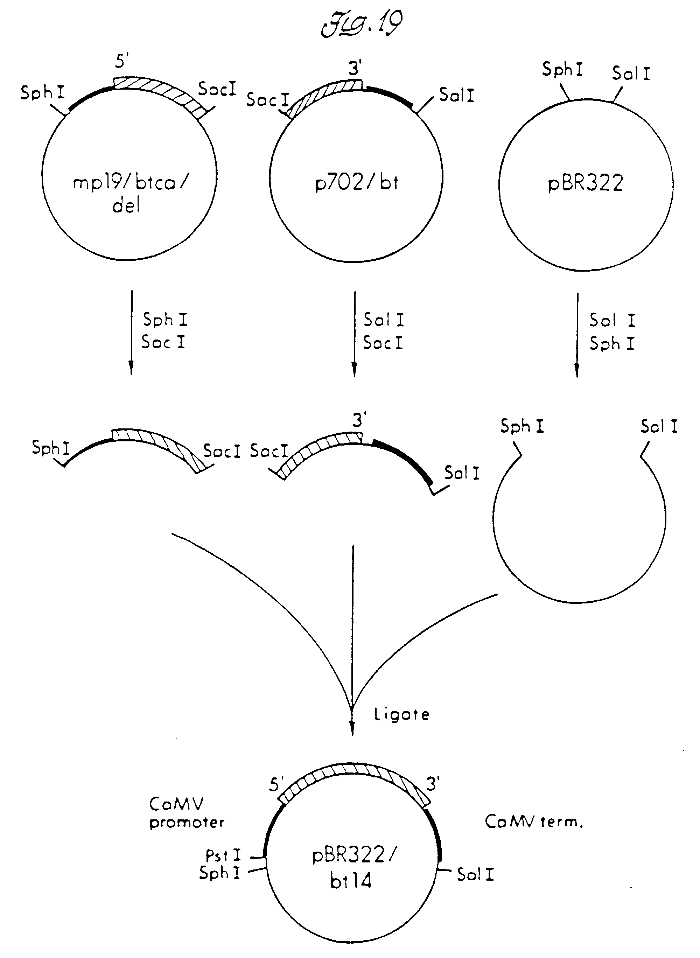

Next, a plasmid was constructed in which the 3' coding region of the protoxin gene was fused to CaMV transcription termination signals. The steps are shown in FIG. 18. First, plasmid pABDI DNA was digested with endonucleases BamHI and BglII and a 0.5 kbp fragment containing the CaMV transcription terminator sequences isolated. Next plasmid pUC19, Yanisch-Perron et al., Gene, 33, 103-119 (1985) incorporated herein by reference was digested with BamHI, mixed with the 0.5 kbp fragment and incubated with $T_4$ DNA ligase. After transformation of the DNA into E. coli strain HB101, one of the resulting clones, called plasmid p702, was obtained which has the structure shown in FIG. 18. Next, plasmid p702 DNA was cleaved with endonucleases SacI and SmaI, and the larger, approximately 3.2 kbp fragment isolated by gel electrophoresis. Plasmid pKU25/4 DNA was digested with endonucleases AhaIII and SacI, and the 2.3-kbp fragment (nucleotides 1502 to 3773 of Formula 1) containing the 3' portion of the protoxin coding sequence (nt 1504 to 3773) was isolated after gel electrophoresis. These two DNA fragments are mixed, incubated with $T_4$ DNA ligase and transformed into E. coli strain HB101. The resulting plasmid was p702/bt (FIG. 18).

Finally, portions of phage mp19/btca/del ds rf DNA and plasmid p702/bt were joined to create a plasmid containing the complete protoxin coding sequence flanked by CaMV promoter and terminator sequences (see FIG. 18). Phage mp19/btca/del DNA was digested with endonucleases SacI and SphI, and a fragment of approx. 1.75 kbp is purified following agarose gel electrophoresis. Similarly, plasmid p702/bt DNA is digested with endonucleases SacI and SalI and a fragment of approximately 2.5 kbp is isolated. Finally, plasmid pBR 322 DNA (Bolivar et al., Gene, 2, 95-113 (1977) incorporated herein by reference was digested with SalI and SphI and the larger 4.2-kbp fragment isolated. All three DNA fragments were mixed and incubated with T4 DNA ligase and transformed into E. coli strain HB101. The resulting plasmid, PBR322/bt14 is a derivative of PBR322 containing the CaMV gene VI promoter and translation start signals fused to the Bacillus thuringiensis crystal protein coding sequence, followed by CaMV transcription termination signals (shown in FIG. 19).

The vector pCIB10 is a Ti-plasmid-derived vector useful for transfer of the chimeric gene to plants via Agrobacterium tumefaciens. The vector is derived from the broad host range plasmid pRK 252, which may be obtained from Dr. W. Barnes, Washington University, St. Louis, Mo. The vector also contains a gene for kanamycin resistance in Agrobacterium, from Tn903, and left and right T-DNA border sequences from the Ti plasmid pTiT37. Between the border sequences are the polylinker region from the plasmid pUC18 and a chimeric gene that confers kanamycin resistance in plants.

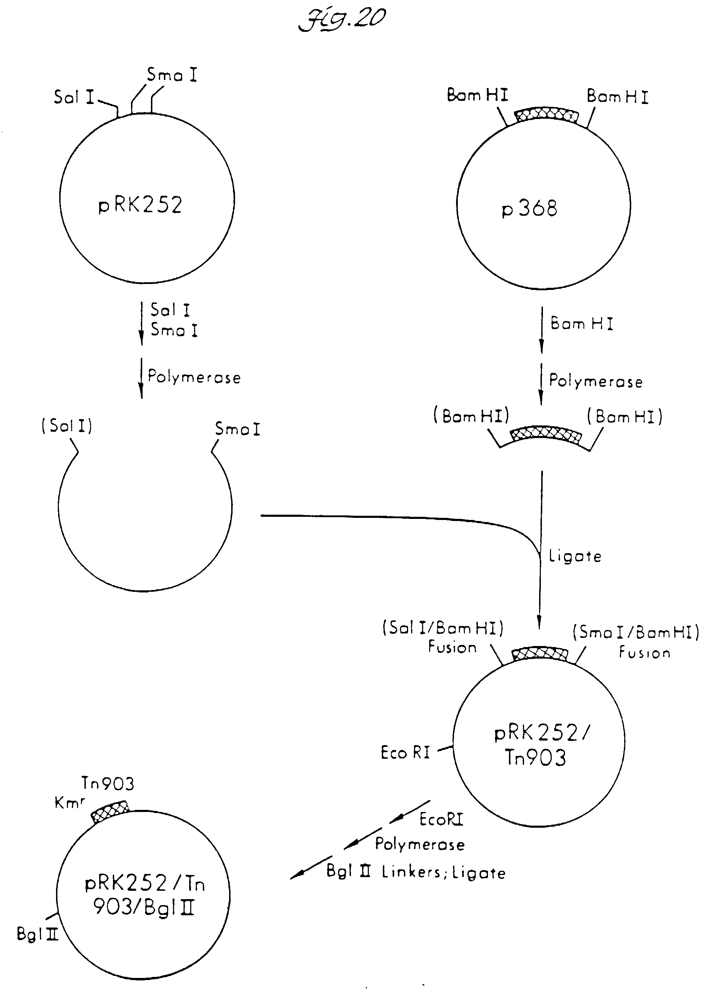

First, plasmid pRK252 was modified to replace the gene conferring tetracycline-resistance with one conferring resistance to kanamycin from the transposon Tn903 [Oka, et al., J. Mol. Biol., 147, 217-226 (1981) incorporated herein by reference], and was also modified by replacing the unique EcoRI site in pRK252 with a BglII site (see FIG. 20 for a summary of these modifications). Plasmid pRK252 was first digested with endonucleases SalI and SmaI, then treated with the large fragment of DNA polymerase I to create flush ends, and the large vector fragment purified by agarose gel electrophoresis. Next, plasmid p368 was digested with endonuclease BamHI, treated with the large fragment of DNA polymerase, and an approximately 1050-bp fragment isolated after agarose gel electrophoresis; this fragment containing the gene from transposon Tn903 which confers resistance to the antibiotic kanamycin [Oka et al., J. Mol. Biol., 147, 217-226 (1981) incorporated herein by reference]. Both fragments were then treated with the large fragment of DNA polymerase to create flush ends. Both fragments are mixed and incubated with T4 DNA ligase overnight at 15°C. After transformation into E. coli strain HB101 and selection for kanamycin resistant colonies, plasmid pRK252/Tn903 is obtained (see FIG. 19).

Plasmid pRK252/Tn903 was digested at its EcoRI site, followed by treatment with the large fragment of E. coli DNA polymerase to create flush ends. This fragment was added to synthetic BglII restriction site linkers, and incubated overnight with $T_4$ DNA ligase. The resulting DNA was digested with an excess of BglII restriction endonuclease and the larger vector fragment purified by agarose gel electrophoresis. The resulting fragment was again incubated with $T_4$ DNA ligase to recircularize the fragment via its newly-added BglII cohesive ends. Following transformation into E. coli strain HB101, plasmid pRK252/Tn903/BglII is obtained (see FIG. 20).

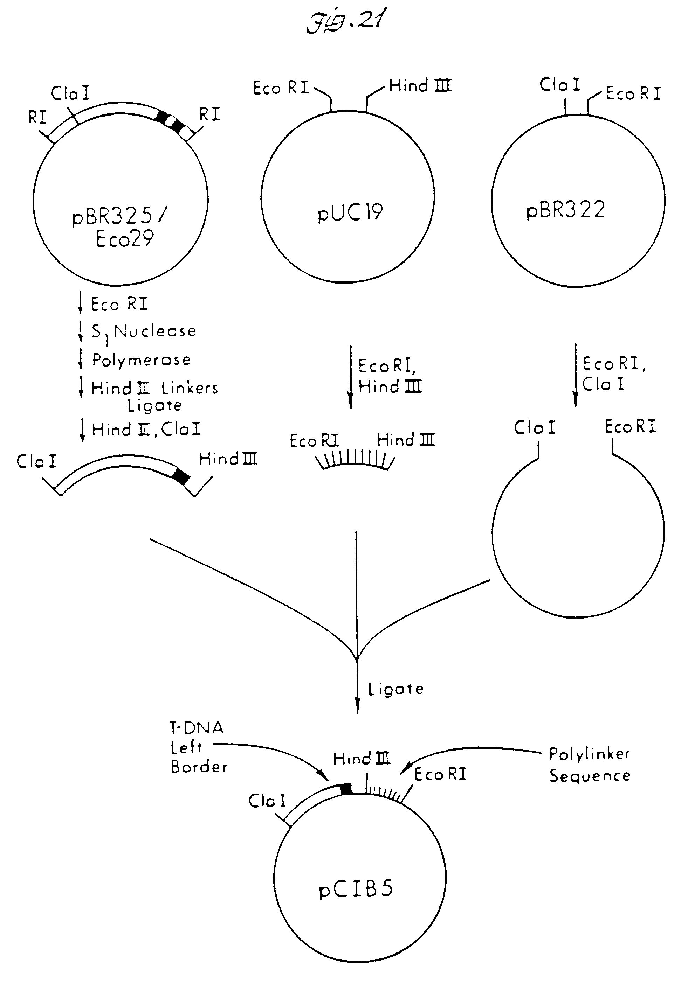

A derivative of plasmid pBR322 was constructed which contains the Ti plasmid T-DNA borders, the polylinker region of plasmid pUC19, and the selectable gene for kanamycin resistance in plants (see FIG. 21). Plasmid pBR325/Eco29 contains the 1.5-kbp EcoRI fragment from the nopaline Ti plasmid pTiT37. This fragment contains the T-DNA left border sequence; Yadav et al., Proc. Natl. Acad. Sci. USA, 79, 6322-6326 (1982) incorporated herein by reference. To replace the EcoRI ends of this fragment with HindIII ends, plasmid pBR325/Eco29 DNA was digested with EcoRI, then incubated with nuclease SI, followed by incubation with the large fragment of DNA polymerase to create flush ends, then mixed with synthetic HindIII linkers and incubated with T4 DNA ligase. The resulting DNA was digested with endonucleases ClaI and an excess of HindIII, and the resulting 1.1-kbp fragment containing the T-DNA left border purified by gel electrophoresis. Next, the polylinker region of plasmid pUC19 was isolated by digestion of the plasmid DNA with endonucleases EcoRI and HindIII and the smaller fragment (approx. 53 bp) isolated by agarose gel electrophoresis. Next, plasmid pBR322 was digested with endonucleases EcoRI and ClaI, mixed with the other two isolated fragments, incubated with T4 DNA ligase and transformed into E. coli strain HB101. The resulting plasmid, pCIB5, contains the polylinker and T-DNA left border in a derivative of plasmid pBR322 (see FIG. 21).

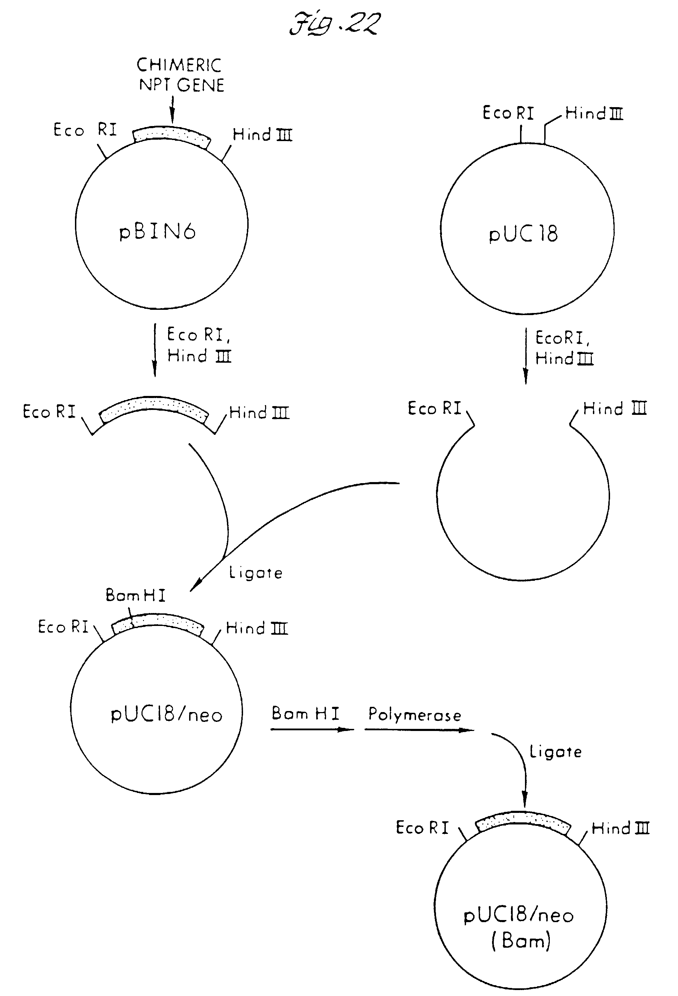

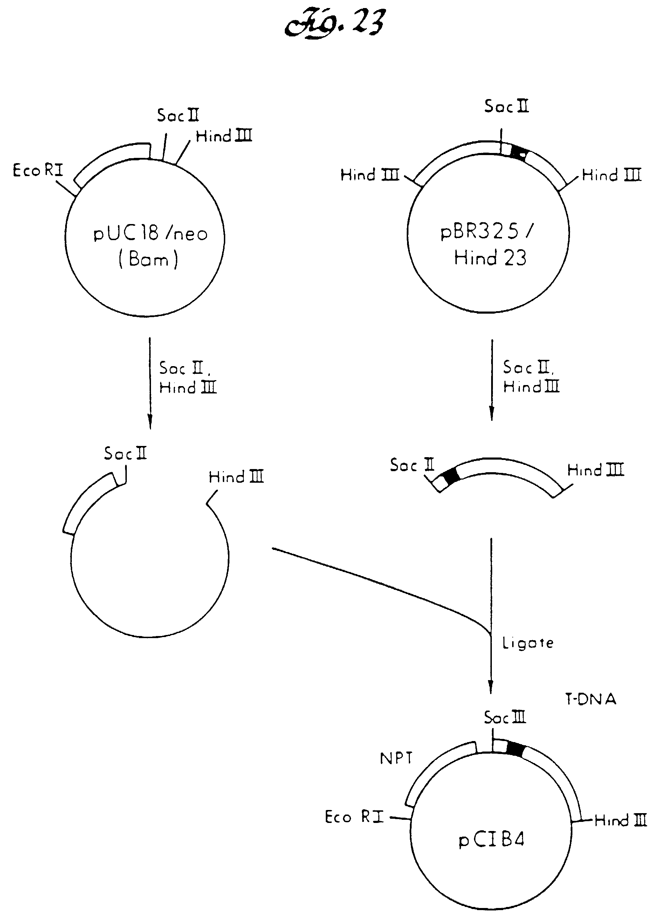

A plasmid containing the gene for expression of kanamycin resistance in plants was constructed (see FIGS. 22 and 23). Plasmid Bin6 obtained from Dr. M. Bevan, Plant Breeding Institute, Cambridge, UK. This plasmid is described in the reference by Bevan, Nucl. Acids Res., 12, 8711-8721 (1984) incorporate herein by reference. Plasmid Bin6 DNA was digested with EcoRI and HindIII and the fragment approximately 1.5 kbp in size containing the chimeric neomycin phosphotransferase (NPT) gene is isolated and purified following agarose gel electrophoresis. This fragment was then mixed with plasmid pUC18 DNA which had been cleaved with endonucleases EcoRI and HindIII. Following incubation with T4 DNA ligase, the resulting DNA was transformed into E. coli strain HB101. The resulting plasmid is called pUC18/neo. This plasmid DNA containing an unwanted BamHI recognition sequence between the neomycin phosphotransferase gene and the terminator sequence for nopaline synthase; see Bevan, Nucl. Acids Res., 12, 8711-8721 (1984) incorporated herein by reference. To remove this recognition sequence, plasmid pUC18/neo was digested with endonuclease BamHI, followed by treatment with the large fragment of DNA polymerase to create flush ends. The fragment was then incubated with T4 DNA ligase to recircularize the fragment, and transformed into E. coli strain HB101. The resulting plasmid, pUC18/neo(Bam) has lost the BamHI recognition sequence.

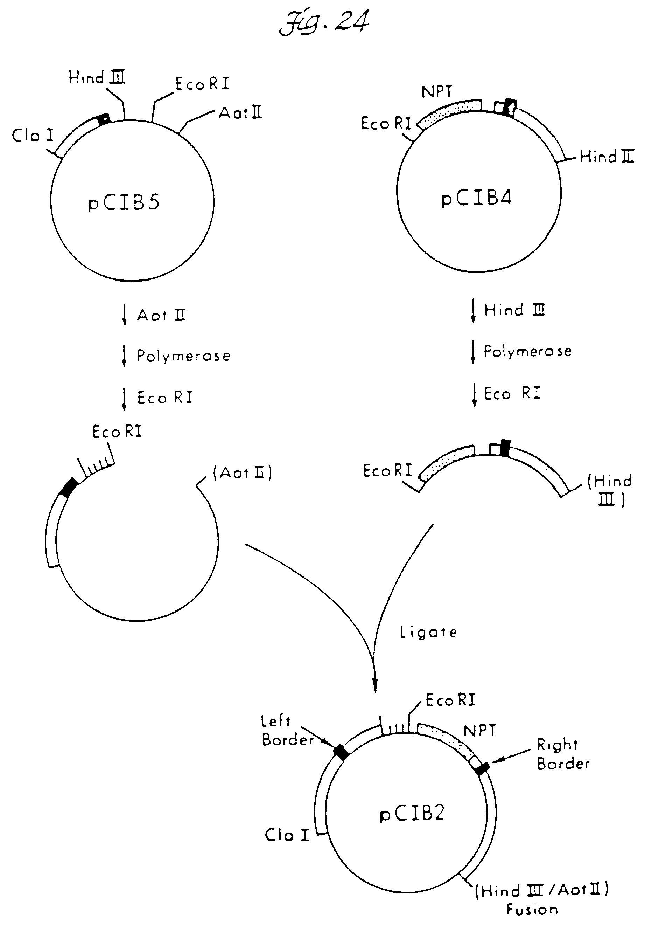

The T-DNA right border sequence was then added next to the chimeric NPT gene (see FIG. 24). Plasmid pBR325/Hind23 contains the 3.4-kbp HindIII fragment of plasmid pTiT37. This fragment contains the right T-DNA border sequence; Bevan et al., Nucl, Acids Res., 11, 369-385 incorporated herein by reference. Plasmid pBR325/Hind23 DNA was cleaved with endonucleases SacII and HindIII, and a 1.0 kbp fragment containing the right border isolated and purified following agarose gel electrophoresis. Plasmid pUC18/neo(Bam) DNA was digested with endonucleases SacII and HindIII and the 4.0 kbp vector fragment isolated by agarose gel electrophoresis. The two fragments were mixed, incubated with T4 DNA ligase and transformed into E. coli strain HB101. The resulting plasmid, pCIB4 (shown in FIG. 23), contains the T-DNA right border and the plant-selectable marker for kanamycin resistance in a derivative of plasmid pUC18.

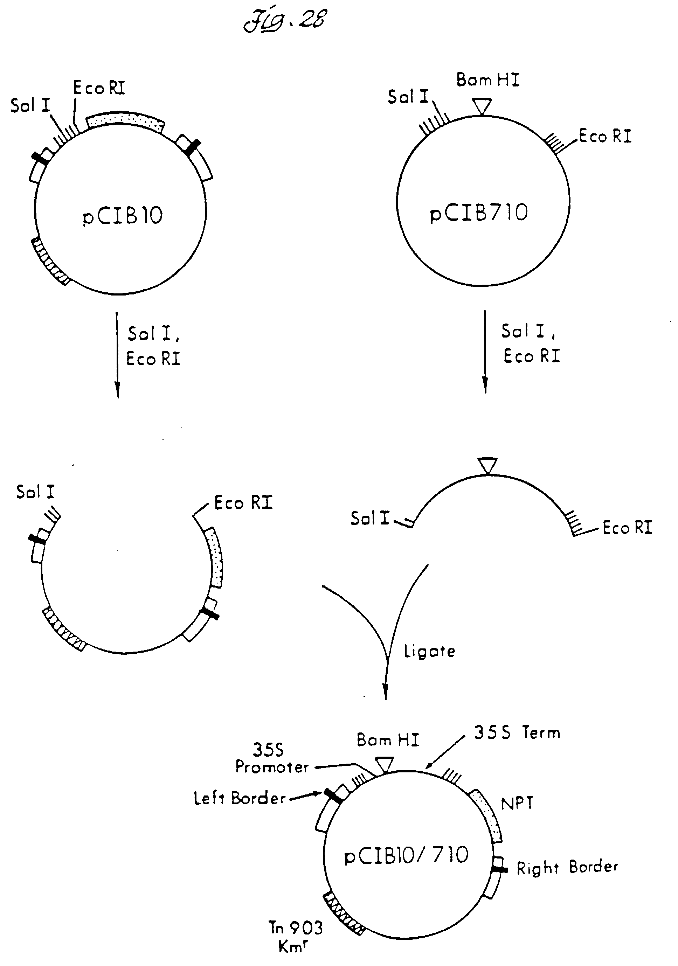

Next, a plasmid was constructed which contains both the T-DNA left and right borders, with the plant selectable kanamycin-resistance gene and the polylinker of pUC18 between the borders (see FIG. 28). Plasmid pCIB4 DNA was digested with endonuclease HindIII, followed by treatment with the large fragment of DNA polymerase to create flush ends, followed by digestion with endonuclease EcoRI. The 2.6-kbp fragment containing the chimeric kanamycin-resistance gene and the right border ot T-DNA was isolated by agarose gel electrophoresis. Plasmid pCIB5 DNA was digested with endonuclease AatII, treated with T4 DNA polymerase to create flush ends, then cleaved with endonuclease EcoRI. The larger vector fragment was purified by agarose gel electrophoresis, mixed with the pCIB4 fragment, incubated with T4 DNA ligase, and transformed into E. coli strain HB101. The resulting plasmid, pCIB2 (shown in FIG. 24) is a derivative of plasmic pBR322 containing the desired sequences between the two T-DNA borders.

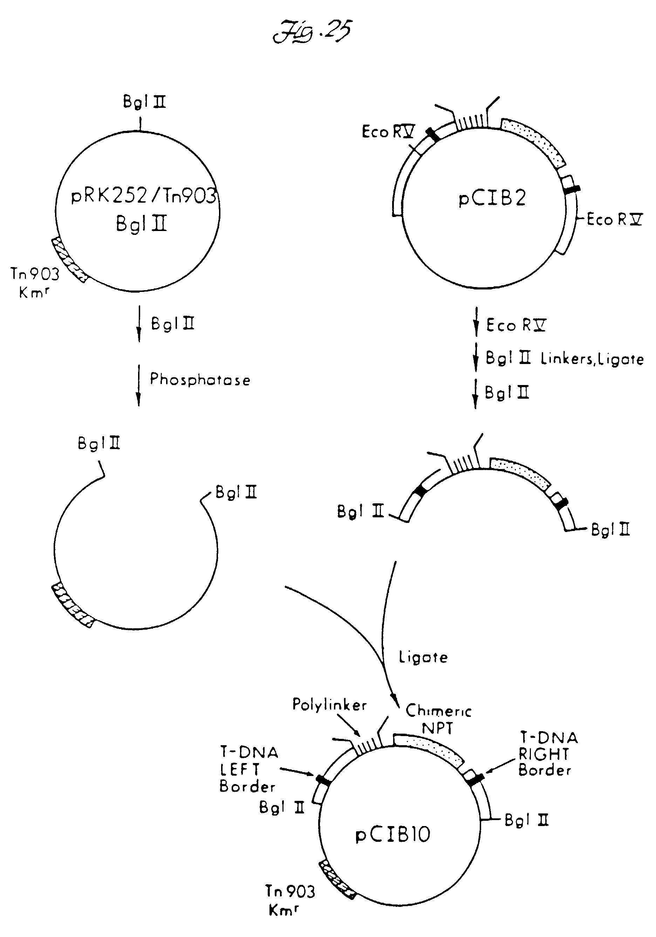

The following steps complete construction of the vector pCIB10, and are shown in FIG. 25. Plasmid pCIB2 DNA was digested with endonuclease EcoRV, and synthetic linkers containing BglII recognition sites are added as described above. After digestion with an excess of BglII endonuclease, the approximately 2.6-kbp fragment was isolated after agarose gel electrophoresis. Plasmid pRK252/Tn903/BglII, described above (see FIG. 20) was digested with endonuclease BglII and then treated with phosphatase to prevent recircularization. These two DNA fragments are mixed, incubated with T4 DNA ligase and transformed into E. coli strain HB101. The resulting plasmid is the completed vector, pCIB10.

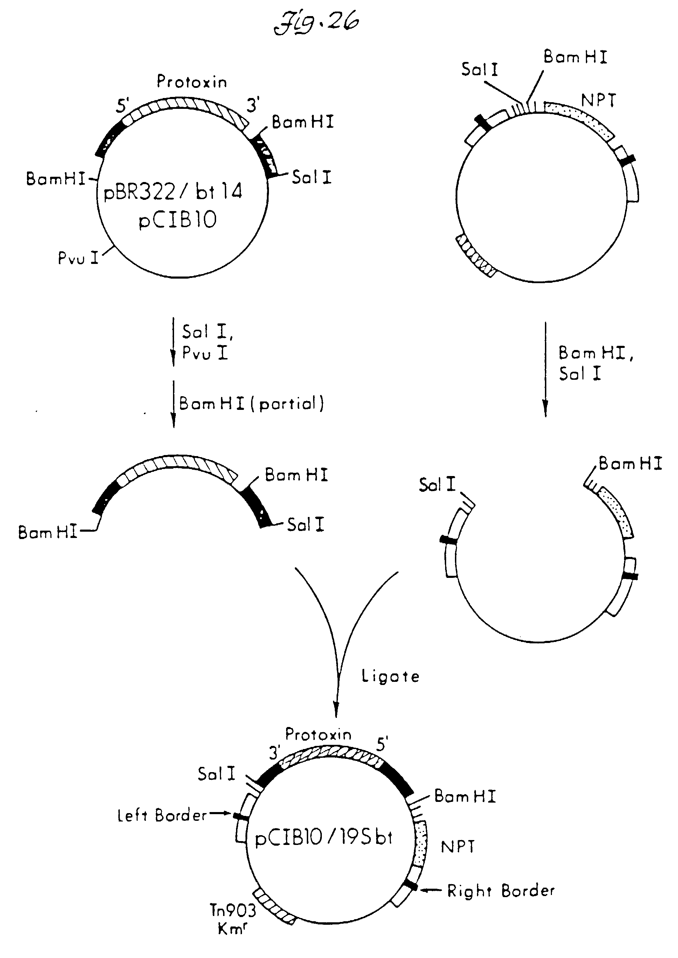

Insertion of the chimeric protoxin gene into vector pCIB10 is by the steps shown in FIG. 26. Plasmid pBR322/bt14 DNA was digested with endonucleases PvuI and SalI, and then partially digested with endonuclease BamHI. A BamHI-SalI fragment approx. 4.2 kbp in size, containing the chimeric gene, was isolated following agarose gel electrophoresis, and mixed with plasmid pCIB10 DNA which had been digested with endonucleases BamHI and SalI. After incubation with T4 DNA ligase and transformation into E. Coli strain HB101, plasmid shown in FIG. 26 and contained the chimeric protoxin gene in the plasmid vector pCIB10.

In order to transfer plasmid pCIB10/19Sbt from E. coli HB101 to Agrobacterium, an intermediate E. coli host strain S17-1 was used. This strain, obtainable from Agrigenetics Research Corp., Boulder, Co. contains mobilization functions that transfer plasmid pCIB10 directly to Agrobacterium via conjugation, thus avoiding the necessity to transform naked plasmid DNA directly into Agrobacterium (reference for strain S17-1 is Simon et al., "Molecular Genetics of the Bacteria-Plant Interaction", A Puhler, ed, Springer Verlag, Berlin, pages 98-106, 1983, incorporated herein by reference). First, plasmid pCIB10/19Sbt DNA is introduced into calcium chloride-treated S17-1 cells. Next, cultures of transformed S17-1 cells and Agrobacteriuam tumefaciens strain LBA4404 [Ooms et al., Gene, 14, 33-50 (1981) incorporated herein by reference] were mixed and mated on an N agar (Difco) plate overnight at room temperature. A loopful of the resulting bacteria are streaked onto AB minimal media; Chilton et al., Proc. Natl. Acad. Sci. USA, 77, 7347-7351 (1974), incorporated herein by reference, plated with 50ug/ml kanamycin and incubated at 28°C. Colonies were restreaked onto the same media, then restreaked onto NB agar plates. Slow-growing colonies were picked, restreaked onto AB minimal media with kanamycin and single colonies isolated. This procedure selects for Agrobacteria containing the pCIB10/19SBt plasmid.

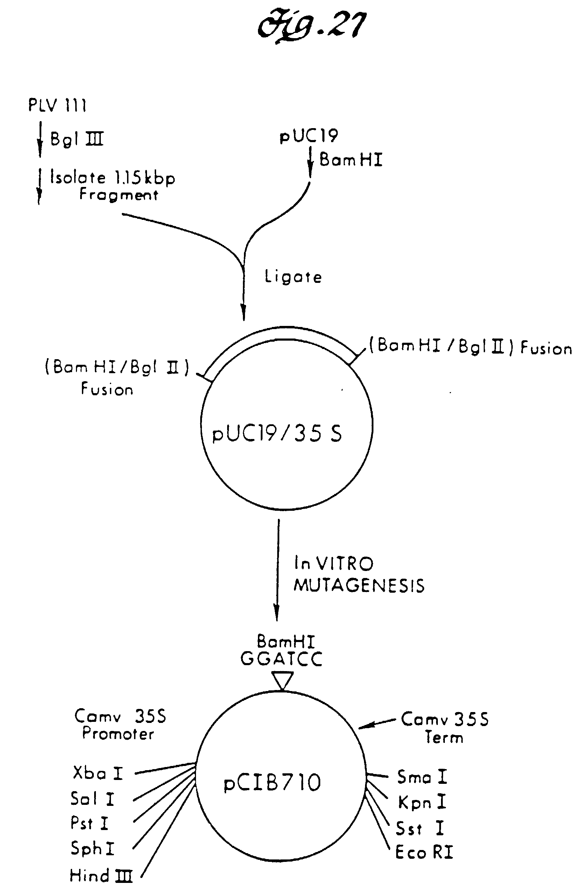

Construction of a Bacillus thuringiensis protoxin chimeric gene with the CaMV 35S promoter was achieved by construction of a CaMV 35S Promoter Cassette Plasmid pCIB710 was constructed as shown in FIG. 27. This plasmid contained CaMV promoter and transcription termination sequences for the 35S RNA transcript [Covey, S.N., Lomonossoff, G.P. and Hull, R., Nucleic Acids Research vol. 9, 6735-6747 (1981) incorporated herein by reference]. A 1149-bp BglII restriction fragment of CaMV DNA in Hohn et al., Current Topics in Microbiology and Immunology, 96, 194-220 and Appendices A to G (1982) incorporated herein by reference] was isolated from plasmid pLVIII (obtained from Dr. S. Howell Univ. California-San Diego; alternatively, the fragment can be isolated directly from CaMV DNA) by preparative agarose gel electrophoresis as described earlier and mixed with BamHI-cleaved plasmid pUC19 DNA, treated with T4 DNA ligase, and transformed into E. coli. The BamHI restriction site in the resulting plasmid has been destroyed by ligation of the BglII cohesive ends to the BamHI cohesive ends. The resulting plasmid, called pUC19/35S, was then used in oligonucleotide-directed in-vitro mutagensis to insert the BamHI recognition sequence GGATCC immediately followingCaMV nucleotide 7483 in the Hohn reference. The resulting plasmid, pCIB710, contains the CaMV 35S promotor region and transcription termination region separated by a BamHI restriction site. DNA sequences inserted into this BamHI site will be expressed in plants by the CaMV transcription regulation sequences. pCIB710 does not contain any ATG translation initiation codons between the start of transcription and the BamHI site.

Insertion of the CaMV 35S promoter/Terminator Cassette into pCIB10 occurred by the steps outlined in FIG. 28. Plasmids pCIB10 and pCIB710 DNAs were digested with EcoRI and SalI, mixed and ligated. The resulting plasmid, pCIB10/710 has the CaMV 35S promoter/terminator cassette inserted into the plant transformation vector pCIB10. The CaMV 35S sequences are between the T-DNA borders in pCIB10, and thus will be inserted into the plant genome in plant transformation.

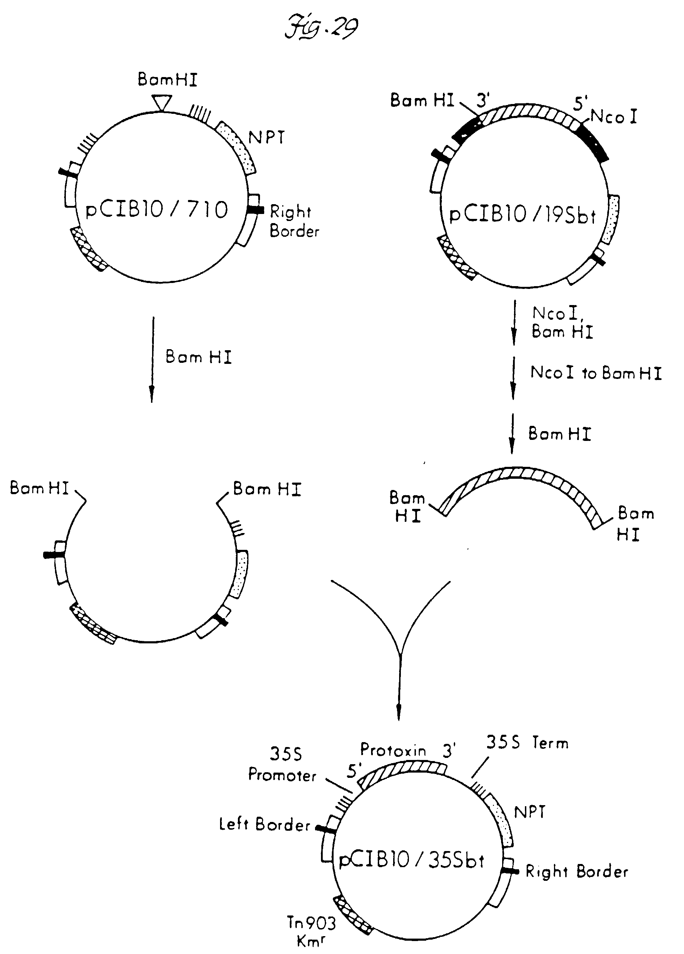

Insertion of the Bacillus thuringiensis protoxin gene into pCIB10/710 occurred by the steps outlined in FIG. 29. As a source of the protoxin gene, plasmid pCIB10/19Sbt was digested with BamHI and Ncol, and the 3.6-kb fragment containing the protoxin gene was isolated by preparative gel electrophoresis. The fragment was then mixed with synthetic Ncol-BamHI adapter with the sequence 5'-CATGGCCGGATCCGGC-3', then digested with BamHI. This step creates BamHI cohesive ends at both ends of the protoxin fragment. This fragment was then inserted into BamHI-cleaved pCIB10/710. The resulting plasmid, pCIB10/35Sbt, shown in FIG. 29, contains the protoxin gene between the CaMV 35S promoter and transcription termination sequences.

Transfer of the plasmid pCIB10/35Sbt into Agrobacterium tumefaciens strain LBA4404 was as described above.

Construction of a deleted Bacillus thuringiensis protoxin gene containing approximately 725 amino acids, and construction of a chimeric gene containing this deleted gene with the CaMV 35S promoter was made by removing the COOH-terminal portion of the gene by cleaving at the KpnI restriction endonuclease site at position 2325 in the sequence shown in Formula 1. Plasmid pCIB10/35Sbt (FIG. 29) was digested with BamHI and KpnI, and the approximately 2.2-kbp BamHI/KpnI fragment containing the deleted protoxin gene isolated by preparative agarose gel electrophoresis. To convert the KpnI site at the 3' end to a BamHI site, the fragment was mixed with a KpnI/BamHI adapter oligonucleotide and ligated. This fragment is then mixed with BamHI-cleaved pCIB10/710 (FIG. 28).

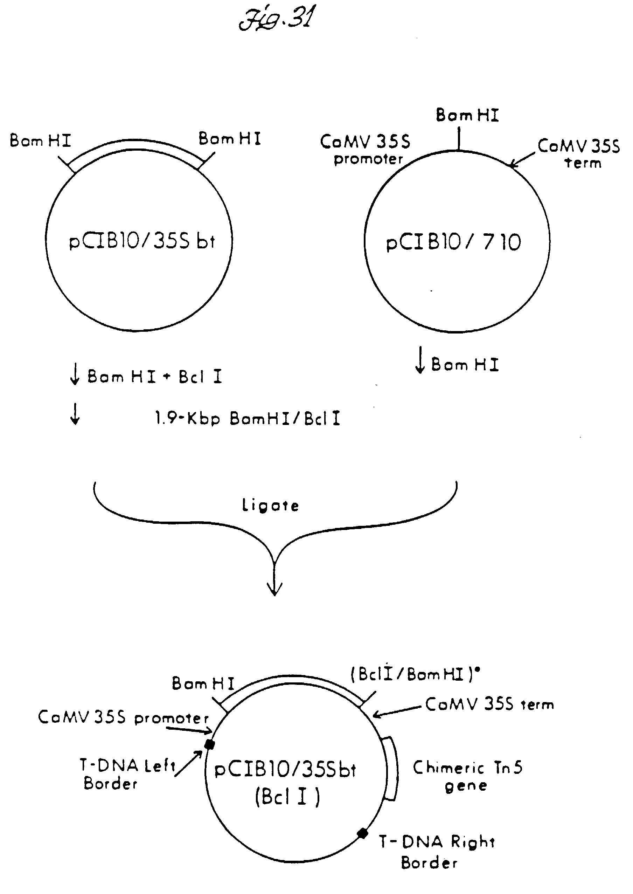

A deleted protoxin gene containing approximately 645 amino acids was made by removing the COOH-terminal portion of the gene by cleaving at the BclI restriction endonuclese site at position 2090 in the sequence shown in Formula 1. Plasmid pCIB10/35Sbt (FIG. 29) was digested with BamHI and BclI, and the approximately 1.9-kbp BamHI/BclI fragment containing the deleted protoxin gene isolated by reprarative agarose gel electrophoresis. Since BclI creates a cohesive end compatible with BamHI, no further manipulation is required prior to ligating this fragment into BamHI-cleaved pCIB10/710 (FIG. 28). The resulting plasmid, which has the structure pCIB10/35Sbt(BclI) shown in FIG. 31 was selected on kanamycin.

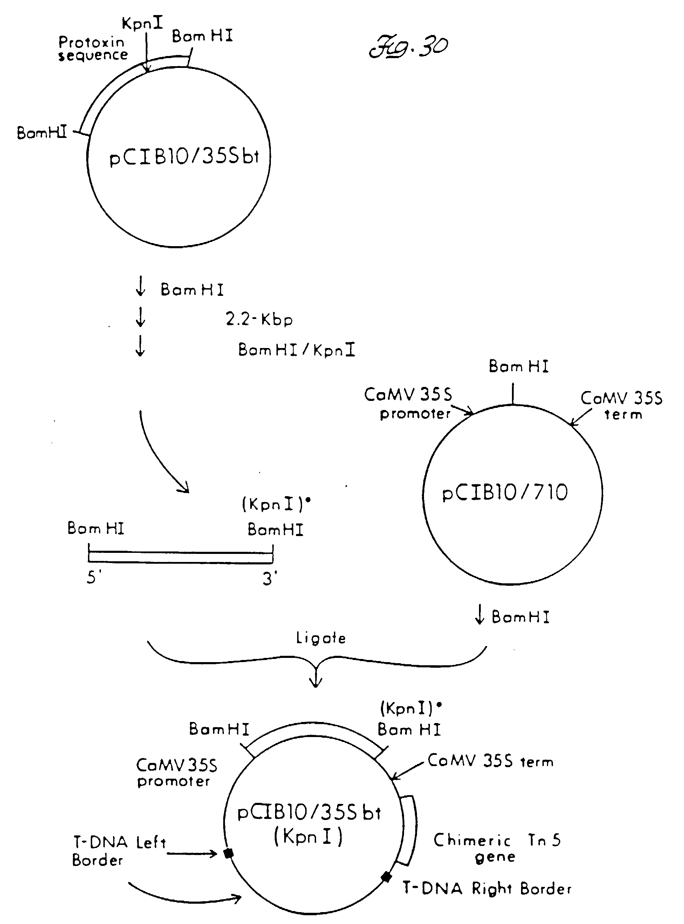

The resulting transformants, designated pCIB10/35Sbt(KpnI) and shown in FIG. 30, contain the deleted protoxin gene of approximately 725 amino acids. These transformants are selected on kanamycin.

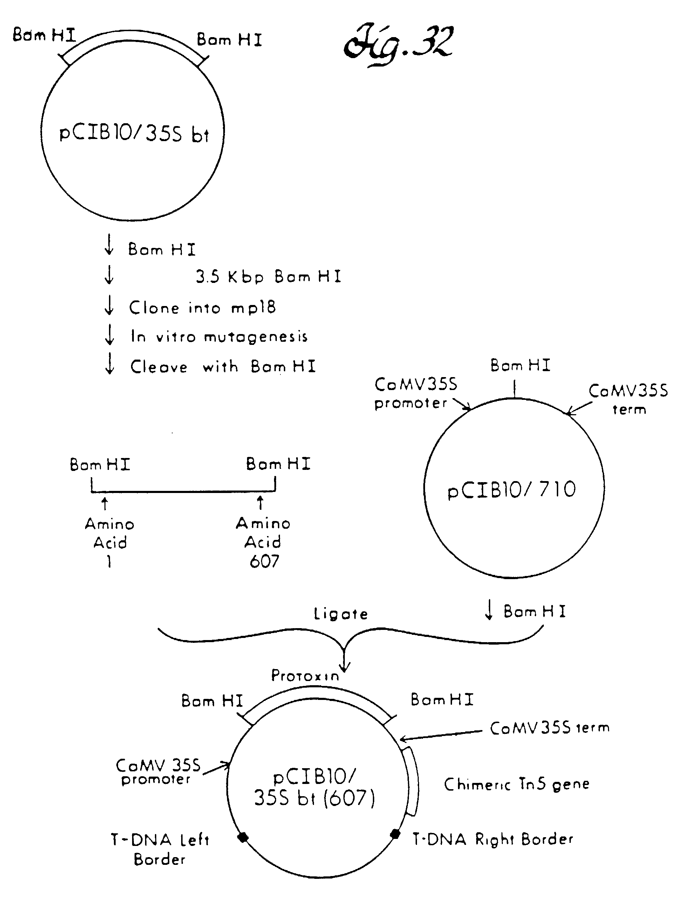

A deleted protoxin gene was made by introducing a BamHI cleavage site (GGATCC). This is done by cloning the BamHI fragment containing the protoxin sequence from pCIB10/35Sbt into mp18, and using standard oligonucleotide mutagensis procedures described above. After mutagensis, double-stranded replicative form DNA is prepared from the M13 clone, which is then digested with BamHI. The approximately 1.9-kbp fragment containing the deleted protoxin gene is inserted into BamHI-cleaved pCIB10/710. The resulting plasmid, which the structure pCIB10/35Sbt(607) shown in FIG. 32 is selected for on kanamycin.

The pCIB10/Sbt 607 was used. Transformation was accomplished as detailed in Example 7 with the change that the 1 ml aliquots were plated immediately on medium containing selective antibiotics. This selection medium contained kanamycin (50 ug/ml) or G418 (25 ug/ml). Expression of the NPT chimeric gene in both transformed plant tissue allows the selection of this tissue on either antibiotic.

In 2-4 weeks, transformed tissue became apparent on the selection plates. Plant material was selected on kanamycin or G418. Plant tissue (either individual embryos or callus) was then extracted with buffer and assayed for expression of the BT gene product by ELISA assay. The conditions of extraction are as follows: per 100mg of tissue, homogenize in 0.1 ml of extraction buffer containing 50 mM $NaCO_3$ (pH9.5), 0.05% Triton, 0.05% Tween, 100nMNaCl, 10mM EDTA, 1mM leupeptine, and 1mM PMSF. The leupeptine and PMSF are added immediately prior to use from 100x stock solutions. The tissue was ground with a motor driven pestle. After extraction, 2M Tris pH7 was added to adjust pH to 8.0-8.5 then centrifuged at 12,000 RPM in a Beckman microfuge 12 (10 minutes at 4°C), and the supernatant saved for enzyme linked immunosorbent assay ("ELISA").

ELISA techniques as a general tool is described by M. F. Clark et al in Methods in Enzymology 118:742-766 (1986), incorporated by reference.

An ELISA for the Bt toxin was developed using standard procedures and used to analyze transgenic plant material for expression of Bt sequences. For this procedure, an ELISA plate is pretreated with ethanol and affinity-purified rabbit anti-Bt antiserum (50 ul) at a concentraction of 3 ug/ml in borate-buffered saline (see below) is added to the plate. This was allowed to incubate overnight at 4°C. Antiserum was produced in response to immunizing rabbits with gradient-purified Bt crystals [Ang, B.J. & Nickerson, K.W.; Appl. Environ. Microbiol. 36: 625-626 (1978)], incorporated herein by reference, solubilized with sodium dodecyl sulfate and washed with ELISA Wash Buffer (see below). It was then treated for 1 hour at room temperature with Blocking Buffer (see below) washed with ELISA Wash Buffer. Plant extract was added in an amount to give 50 ug of protein (this is typically ca. 5 microliters of extract). Leaf extraction buffer as protein is determined by the Bradford method [Bradford, M., Anal. Biochem. 72:248 (1976) incorporated herein by reference] using a commercially available kit obtained from Bio-Rad, Richmond, California. If dilution of the leaf extract is necessary, ELISA Diluent (see below)] isused. Allow this to incubate overnight at 4°C. After a wash with ELISA Wash Buffer, 50 ul affinity-purified goat anti-Bt antiserum is added at a concentration of 3 ug/ml protein in ELISA Diluent. This is allowed to incubate for 1 hour at 37°C, then washed with ELISA Wash Buffer. 50 ul rabbit anti-goat antibody bound to alkaline phosphatase [commercially available from Sigma Chemicals, St. Louis, Mo.] is diluted 1:500 in ELISA Diluent and allowed to incubate for 1 hour at 37°C, then washed with ELISA Wash Buffer. 50 microliters substrate [0.6 mg/ml p-nitrophenyl phosphate in ELISA Substrate Buffer (see below) are added and incubated for 30 minutes at room temperature. Reaction is terminated by adding 50 microliters of 3 M NaOH. Absorbance is read at 405 nm in modified ELISA reader [Hewlett Packard, Stanford, Ca.].

Plant tissue transformed with the pCIB10/35SBt(BcII) when assayed using this ELISA procedure showed a positive reaction, indicating expression of the Bt gene.

| EPBS (ELISA Phosphate Buffered Saline) | | |
|---|---|---|
| 10 mM NaPhosphate: | $Na_2HPO_4$ | 4.68 grams/4 liters |
| | $NaH_2PO_4.H2O$ | 0.976 grams/4 liters |
| 140 mM NaCl | NaCl | 32.7 grams/4 liters |
| pH should be approximately 7.4 | | |

Borate Buffered Saline

100 mM Boric acid
25 mM Na Borate
75 mM NaCl

Adjust pH to 8.4-8.5 with HCl or NaOH as needed.

ELISA Blocking Buffer

In EPBS,

1% BSA
0.02% Na azide

ELISA Wash Buffer

10mM Tris-HCl pH 8.0
0.05% Tween 20
0.02% Na Azide

2.5M TRIS

ELISA Diluent

In EPBS:

0.05% Tween 20
1% BSA
0.02% Na Azide

ELISA Substrate Buffer

In 500 mls,

48 ml Diethanolamine,
24.5 mg MgCl$_2$;

adjust to pH 9.8 with HCl.

ELISA Substrate

15 mg p-nitrophenyl phosphate in 25 ml Substrate Buffer.

For bioassays, cell suspensions from antibioticresistant cell cultures obtained from transformations with these Agrobacteria were initiated. Suspensions were grown in medium supplemented with G418 (25mg/L), and subcultured into fresh antibiotic-containing medium on 7-10 day intervals. Samples of these cultures were then used in bioassays to test for toxicity to lepidopterous insects. Twenty ml aliquots of these cultures were allowed to settle (cell volume = 3-4ml), and resuspended in medium lacking antibiotics. Suspensions were then allowed to grow for an additional two days in this medium to deplete the cells of any residual antibiotic. Two circles of wet Whatman 2.3 cm filter paper were placed in the bottom of a 3/4 oz portion cup. A layer of transformed suspension culture cells 0.2 cm deep was placed onto the filter paper disk. A newly-hatched Manduca sexta or Heliothis virescens larva was placed into each portion cup. Controls were made up of larvae fed on non-transformed suspension culture cells. Discs were replenished on 2-day intervals or as needed. Manduca larvae generally require more plant material. The growth rate and mortality of the larvae feeding on transformed cells compared with the growth rate of larvae feeding on untransformed cells was scored after 5 days, and clearly affirmed the toxicity of the BT gene product in transformed cotton cells.

EXAMPLE 15

Heliothis virescens eggs laid on sheets of cheesecloth are obtained from the Tobacco Insect Control Laboratory at North Carolina State University, Raleigh, North Carolina. The cheesecloth sheets are transferred to a large covered glass beaker and incubated at 29 degrees C with wet paper towels to maintain humidity. The eggs hatched within three days. As soon as possible after hatching, the larvae (one larva per cup) are transferred to covered 3/4 oz. plastic cups. Each cup contains cotton leaf discs. Larvae are transferred using a fine bristle paint brush.

Leaf discs one centimeter in diameter are punched from leaves of cotton plants and placed on a circle of wet filter paper in the cup with the larva. At least 6-10 leaf discs, representing both young and old leaves, are tested from each plant. Leaf discs are replaced at two-day intervals, or as necessary to feed the larvae. Growth rates [size or combined weight of all replica worms] and mortality of larvae feeding on leaves of transformed plants are compared with those of larva feeding on untransformed cotton leaves.

Larvae feeding on discs of cotton transformed with pCIB10/35SB4 (BcII) show a decrease in growth rate and increase in mortality compared with controls.

It was observed that a certain number of our regenerated plants (5-10%) appeared to have acquired genetically heritable phenotypic variations as a consequence of the process of regeneration. This variation is known as somaclonal variation. The following examples illustrate how somaclonal variation as a consequence of our regeneration procedure has been used to introduce commercially useful new traits into cotton varieties.

EXAMPLE 16

Cotton Regenerants Tolerant to Fungal Pathogens

The procedure of Example 1 was followed, and regenerated cotton plants obtained of the variety SJ5 and SJ4 were hardened and placed in the soil. These plants were self-pollinated and the seed, representing the F1 generation, collected.

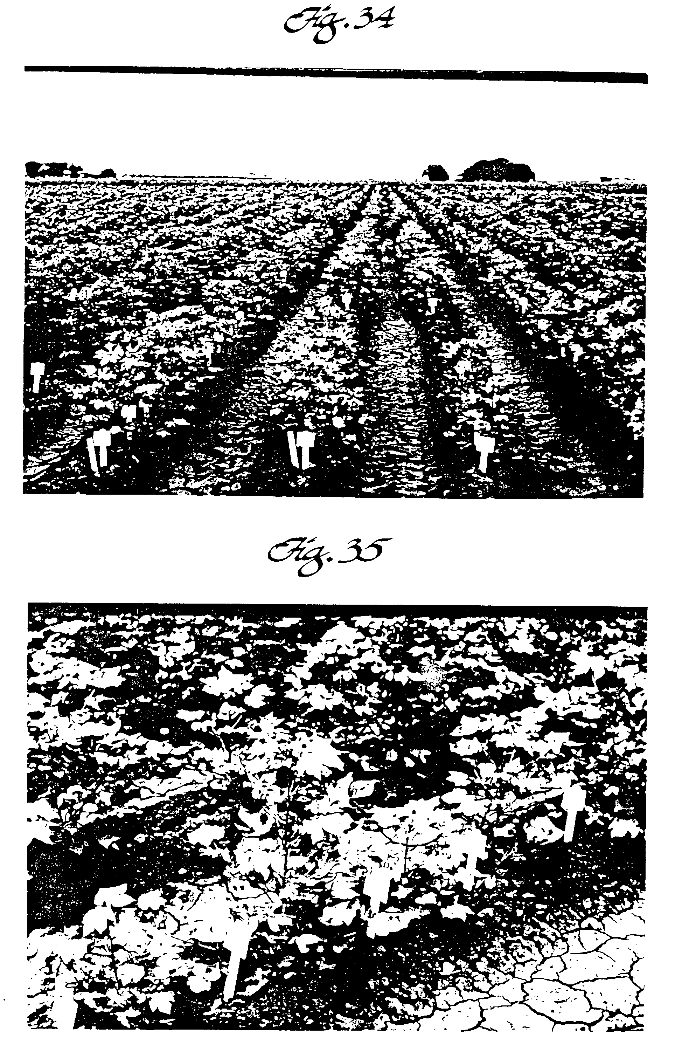

To obtain regenerants (somaclonal variants) more tolerant to Verticillium, the F1 generation was planted in a Verticillium infested field for progeny row analysis. Seed of the varieties SJ4 and SJ5 were planted in the field as controls. Somaclonal variants more tolerant than the parental varieties to the Verticillium fungus were identified in a few of the progeny rows (5%) by assessing overall plant vigor, yield, and the absence of foliar symtoms associated with the disease. FIG. 33 shows the progeny rows of regenerants planted in a Verticillium infested field. FIG. 34 shows a Verticillium tolerant somaclonal variant of variety SJ4. This improvement in tolerance to the fungal pathogen was found to be genetically stable and passed on to subsequent generations.

EXAMPLE 17

Cotton Regenerants with altered growth habits

The procedure of Example 13 was followed except that, rather than planting in disease-infested soil, the F1 generation was planted in a cotton breeding nursery. The overall growth habit of the F1 regenerated progeny was compared to that of the control varieties. Somaclonal variants were identified which were more uniform in growth habit and shorter in stature than the parental variety. One SJ5 regenerant, identified in our trials as Phy 6, was 20% shorter in stature than the parental variety. This kind of growth habit is desireable in cotton grown under narrow row (30" row spacing) cultural conditions. These traits were found to be genetically stable and passed on to subsequent generations.

EXAMPLE 18

Cotton regenerants with improved fiber traits

The procedure of Example 13 was followed except that the F1 progeny of regenerants were planted in a cotton breeding nursery and allowed to set fruit. When the bolls were mature, the cotton was harvested and subjected to an analysis of several fiber quality traits including length, uniformity, tensile strength, elasticity, and micronaire. Somaclonal variants were identified which were improved significantly over the parental variety in one or more of these traits. Representative data from F2 progeny (cell pollination of the F1) are included in the following Table 1. Values marked with an asterisk represent improvements in SJ5 regenerants which are statistically significant and have been found to breed true in subsequent generations.

Table 1

| Variety or strain | Length | Uniformity Index | Fiber Properties | | |
|---|---|---|---|---|---|
| | | | Tensile Strength | Elasticity | Variety or strain |
| SJ5 | 1.13 | 48.7 | 24.7 | 6.8 | 4.27 |
| 3SP16 | 1.27* | 51.2 | 24.6 | 8.0* | 4.10* |
| 3SP20 | 1.28* | 53.1* | 23.1 | 7.6* | 4.13* |
| 5SP10 | 1.11 | 53.2* | 25.7* | 6.2 | 4.55 |
| 5SP17 | 1.18 | 51.7 | 26.7* | 7.1 | 4.43 |

EXAMPLE 19

Cotton regenerants with improved yield

The procedure of Example 13 was followed except that the F1 progeny of regenerants of the variety SJ4 were planted in replicated yield trials along with nonregenerated controls. One variant, which exhibited a more uniform growth habit and more vigorous growth habit, yielded 4% more cotton than the parental variety in the same trial. The data are given in Table 2 below.

Table 2

| Variety or Strain | $\overline{X}$ Yield per plot (lb) | $\overline{X}$ Yield lbs/Acre | % Increase |
|---|---|---|---|
| SJ4 Control | 28.0 | 3049 | |
| Phy 4 | 29.1 | 3169 | 4%* |

*This difference was significant at the 95% confidence level.

A 4% increase in yield would represent a return of almost $20 per acre to the average cotton grower in California, where over one million acres of cotton are grown annually.

EXAMPLE 20

Cotton Regenerants tolerant to a herbicide. (kanamycin)

Suspension cultures of the cotton variety B1644 were developed according to the method of Example 5. Suspension cultures were then plated onto an agar medium as described in Example 6, but supplemented with the herbicide (antibiotic) kanamycin (25mg/l). Most of the cells in the population died, but a few (1 to 5%) were tolerant and survived. These were selectively subcultured onto agar-solidified media supplemented with increasing concentrations of kanamycin, until the final concentration reached 50mg/l. Embryos were then developed from this callus, and those resistant embryos were germinated into kanamycin resistant plants.

EP 0 344 302 B1

## FORMULA I

```
        · 10        20       · 30       ·  40          50         60
GTTAACACCC TGGGTCAAAA ATTGATATTT AGTAAAATTA GTTGCACTTT GTGCATTTTT

        70         80       · 90         100  ·      110        120
TCATAAGATG AGTCATATGT TTTAAATTGT AGTAATGAAA AACAGTATTA TATCATAATG

       130  ·      140        150        160        170        180
AATTGGTATC TTAATAAAAG AGATGGAGGT AACTTATGGA TAACAATCCG AACATCAATG

       190  ·      200        210        220        230        240
AATGCCATTCC TTATAATTGT TTAAGTAACC CTGAAGTAGA AGTATTAGGT GGAGAAAGAA

       250        260        270       · 280        290        300
TAGAAACTGG TTACACCCCA ATCGATATTT CCTTGTCGCT AACGCAATTT CTTTTGAGTG

       310        320        330        340        350        360
AATTTGTTCC CGGTGCTGGA TTTGTGTTAG GACTAGTTGA TATAATATGG GGAATTTTTG

       370        380        390        400        410        420
GTCCCTCTCA ATGGGACGCA TTTCCTGTAC AAATTGAACA GTTAATTAAC CAAAGAATAG

       430        440        450        460        470        480
AAGAATTCGC TAGGAACCAA GCCATTTCTA GATTAGAAGG ACTAAGCAAT CTTTATCAAA

       490        500        510        520        530        540
TTTACGCAGA ATCTTTTAGA GAGTGGGAAG CAGATCCTAC TAATCCAGCA TTAAGAGAAG

       550        560       · 570        580        590        600
AGATGCGTAT TCAATTCAAT GACATGAACA GTGCCCTTAC AACCGCTATT CCTCTTTTTG

     · 610        620      · 630       · 640        650        660
CAGTTCAAAA TTATCAAGTT CCTCTTTTAT CAGTATATGT TCAAGCTGCA AATTTACATT

       670        680        690        700        · 710        720
TATCAGTTTT GAGAGATGTT TCAGTGTTTG GACAAAGGTG GGGATTTGAT GCCGCGACTA

       730        740        750        760        770        780
TCAATAGTCG TTATAATGAT TTAACTAGGC TTATTGGCAA CTATACAGAT CATGCTGTAC

       790        800        810        820        830        840
GCTGGTACAA TACGGGATTA GAGCGTGTAT GGGGACCGGA TTCTAGAGAT TGGATAAGAT

       850        860        870        880        890        900
ATAATCAATT TAGAAGAGAA TTAACACTAA CTGTATTAGA TATCGTTTCT CTATTTCCGA

       910        920        930        940        950        960
ACTATGATAG TAGAACGTAT CCAATTCGAA CAGTTTCCCA ATTAACAAGA GAAATTTATA

       970        980        990        1000       1010       1020
CAAACCCAGT ATTAGAAAAT TTTGATGGTA GTTTTCGAGG CTCGGCTCAG GGCATAGAAG

      1030       1040       1050       1060       1070       1080
GAAGTATTAG GAGTCCACAT TTGATGGATA TACTTAACAG TATAACCATC TATACGGATG

      1090       1100       1110       · 1120       · 1130       1140
CTCATAGAGG AGAATATTAT TGGTCAGGGC ATCAAATAAT GGCTTCTCCT GTAGGGTTTT

      1150       1160       1170       · 1180       1190       1200
CGGGGCCAGA ATTCACTTTT CCGCTATATG GAACTATGGG AAATGCAGCT CCACAACAAC

      1210       1220       1230       1240       1250       1260
GAATTGTTGC TCAACTAGGT CAGGGCGTGT ATAGAACATT ATCGTCCACT TTATATAGAA
```

24

```
        1270         1280         1290         1300         1310         1320
GACCT.TTTAA TATAGGGATA AATAATCAAC AACTATCTGT TCTTGACGGG ACAGAATTTG

        1330         1340         1350         1360         1370         1380
CTTATGGAAC CTCCTCAAAT TTGCCATCCG CTGTATACAG AAAAAGCGGA ACGGTAGATT

        1390         1400         1410         1420         1430         1440
CGCTGGATGA AATACCGCCA CAGAATAACA ACGTGCCACC TAGGCAAGGA TTTAGTCATC

        1450         1460         1470         1480         1490         1500
GATTAAGCCA TGTTTCAATG TTTCGTTCAG GCTTTAGTAA TAGTAGTGTA AGTATAATAA

        1510         1520         1530         1540         1550         1560
GAGCTCCTAT GTTCTCTTGG ATACATCGTA GTGCTGAATT TAATAATATA ATTCCTTCAT

        1570         1580         1590         1600         1610         1620
CACAAATTAC ACAAATACCT TTAACAAAAT CTACTAATCT TGGCTCTGGA ACTTCTGTCG

        1630         1640         1650         1660         1670         1680
TTAAAGGACC AGGATTTACA GGAGGAGATA TTCTTCGAAG AACTTCACCT GGCCAGATTT

        1690         1700         1710         1720         1730         1740
CAACCTTAAG AGTAAATATT ACTGCACCAT TATCACAAAG ATATCGGGTA AGAATTCGCT

        1750         1760         1770         1780         1790         1800
ACGCTTCTAC CACAAATTTA CAATTCCATA CATCAATTGA CGGAAGACCT ATTAATCAGG

        1810         1820         1830         1840         1850         1860
CGAATTTTTC AGCAACTATG AGTAGTGGGA GTAATTTACA GTCCGGAAGC TTTAGGACTG

        1870         1880         1890         1900         1910         1920
TAGGTTTTAC TACTCCGTTT AACTTTTCAA ATGGATCAAG TGTATTTACG TTAAGTGCTC

        1930         1940         1950         1960         1970         1980
ATGTCTTCAA TTCAGGCAAT GAAGTTTATA TAGATCGAAT TGAATTTGTT CCGGCAGAAG

        1990         2000         2010         2020         2030         2040
TAACCTTTGA GGCAGAATAT GATTTAGAAA GAGCACAAAA GGCGGTGAAT GAGCTGTTTA

        2050         2060         2070         2080         2090         2100
CTTCTTCCAA TCAAATCGGG TTAAAAACAG ATGTGACGGA TTATCATATT GATCAAGTAT

        2110         2120         2130         2140         2150         2160
CCAATTTAGT TGAGTGTTTA TCTGATGAAT TTTGTCTGGA TGAAAAAAAA GAATTGTCCG

        2170         2180         2190         2200         2210         2220
AGAAAGTCAA ACATGCGAAG CGACTTAGTG ATGAGCGGAA TTTACTTCAA GATCCAAACT

        2230         2240         2250         2260         2270         2280
TTAGAGGGAT CAATAGAGAA CTAGACCGTG GCTGGAGAGG AAGTACGGAT ATTACCATCC

        2290         2300         2310         2320         2330         2340
AAGGAGGCGA TGACGTATTC AAAGAGAATT ACGTTACGCT ATTGGGTACC TTTGATGAGT

        2350         2360         2370         2380         2390         2400
GCTATCCAAC GTATTTATAT CAAAAAATAG ATGAGTCGAA ATTAAAAGCC TATACCCGTT

        2410         2420         2430         2440         2450         2460
ACCAATTAAG AGGGTATATC GAAGATAGTC AAGACTTAGA AATCTATTTA ATTCGCTACA

        2470         2480         2490         2500         2510         2520
ATGCCAAACA CGAAACAGTA AATGTGCCAG GTACGGGTTC CTTATGGCCG CTTTCAGCCC
```

```
        2530        2540        2550        2560        2570        2580
CAAGTCCAAT CGGAAAATGT GCCCATCATT CCCATCATTT CTCCTTGGAC ATTGATGTTG

        2590        2600        2610        2620        2630        2640
GATGTACAGA CTTAAATGAG GACTTAGGTG TATGGGTGAT ATTCAAGATT AAGACGCAAG

        2650        2660        2670        2680        2690        2700
ATGGCCATGC AAGACTAGGA AATCTAGAAT TTCTCGAAGA GAAACCATTA GTAGGAGAAG

        2710        2720        2730        2740        2750        2760
CACTAGCTCG TGTGAAAAGA GCGGAGAAAA AATGGAGAGA CAAACGTGAA AAATTGGAAT

        2770        2780        2790        2800        2810        2820
GGGAAACAAA TATTGTTTAT AAAGAGGCAA AAGAATCTGT AGATGCTTTA TTTGTAAACT

        2830        2840        2850        2860        2870        2880
CTCAATATGA TAGATTACAA GCGGATACCA ACATCGCGAT GATTCATGCG GCAGATAAAC

        2890        2900        2910        2920        2930        2940
GCGTTCATAG CATTCGAGAA GCTTATCTGC CTGAGCTGTC TGTGATTCCG GGTGTCAATG

        2950        2960        2970        2980        2990        3000
CGGCTATTTT TGAAGAATTA GAAGGGCGTA TTTTCACTGC ATTCTCCCTA TATGATGCGA

        3010        3020        3030        3040        3050        3060
GAAATGTCAT TAAAAATGGT GATTTTAATA ATGGCTTATC CTGCTGGAAC GTGAAAGGGC

        3070        3080        3090        3100        3110        3120
ATGTAGATGT AGAAGAACAA AACAACCACC GTTCGGTCCT TGTTGTTCCG GAATGGGAAG

        3130        3140        3150        3160        3170        3180
CAGAAGTGTC ACAAGAAGTT CGTGTCTGTC CGGGTCGTGG CTATATCCTT CGTGTCACAG

        3190        3200        3210        3220        3230        3240
CGTACAAGGA GGGATATGGA GAAGGTTGCG TAACCATTCA TGAGATCGAG AACAATACAG

        3250        3260        3270        3280        3290        3300
ACGAACTGAA GTTTAGCAAC TGTGTAGAAG AGGAAGTATA TCCAAACAAC ACGGTAACGT

        3310        3320        3330        3340        3350        3360
GTAATGATTA TACTGCGACT CAAGAAGAAT ATGAGGGTAC GTACACTTCT CGTAATCGAG

        3370        3380        3390        3400        3410        3420
GATATGACGG AGCCTATGAA AGCAATTCTT CTGTACCAGC TGATTATGCA TCAGCCTATG

        3430        3440        3450        3460        3470        3480
AAGAAAAAGC ATATACAGAT GGACGAAGAG ACAATCCTTG TGAATCTAAC AGAGGATATG

        3490        3500        3510        3520        3530        3540
GGGATTACAC ACCACTACCA GCTGGCTATG TGACAAAAGA ATTAGAGTAC TTCCCAGAAA

        3550        3560        3570        3580        3590        3600
CCGATAAGGT ATGGATTGAG ATCGGAGAAA CGGAAGGAAC ATTCAACGTG GACAGCGTGG

        3610        3620        3630        3640        3650        3660
AATTACTTCT TATGGAGGAA TAATATATGC TTTATAATGT AAGGTGTGCA AATAAAGAAT

        3670        3680        3690        3700        3710        3720
GATTACTGAC TTGTATTGAC AGATAAATAA GGAAATTTTT ATATGAATAA AAAACGGGCA

        3730        3740        3750        3760        3770        3780
TCACTCTTAA AAGAATGATG TCCGTTTTTT GTATGATTTA ACGAGTGATA TTTAAATGTT
```

```
      3790       3800       3810       3820       3830       3840
TTTTTTGCGA AGGCTTTACT TAACGGGGTA CCGCCACATG CCCATCAACT TAAGAATTTG

      3850       3860       3870       3880       3890       3900
CACTACCCCC AAGTGTCAAA AAACGTTATT CTTTCTAAAA AGCTAGCTAG AAAGGATGAC

      3910       3920       3930       3940       3950       3960
ATTTTTTATG AATCTTTCAA TTCAAGATGA ATTACAACTA TTTTCTGAAG AGCTGTATCG

      3970       3980       3990       4000       4010       4020
TCATTTAACC CCTTCTCTTT TGGAAGAACT CGCTAAAGAA TTAGGTTTTG TAAAAAGAAA

      4030       4040       4050       4060       4070       4080
ACGAAAGTTT TCAGGAAATG AATTAGCTAC CATATGTATC TGGGGCAGTC AACGTACAGC

      4090       4100       4110       4120       4130       4140
GAGTGATTCT CTCGTTCGAC TATGCAGTCA ATTACACGCC GCCACAGCAC TCTTATGAGT

      4150       4160       4170       4180       4190       4200
CCAGAAGGAC TCAATAAACG CTTTGATAAA AAAGCGGTTG AATTTTTGAA ATATATTTTT

      4210       4220       4230       4240       4250       4260
TCTGCATTAT GGAAAAGTAA ACTTTGTAAA ACATCAGCCA TTTCAAGTGC AGCACTCACG

      4270       4280       4290       4300       4310       4320
TATTTTCAAC GAATCCGTAT TTTAGATGCG ACGATTTTCC AAGTACCGAA ACATTTAGCA

      4330       4340       4350       4360
CATGTATATC CTGGGTCAGG TGGTTGTGCA CAAACTGCAG
```

## Claims

1. A method for the regeneration of a cotton plant from somatic cells which comprises the steps of:

   a) providing a cotton explant;

   b) culturing the explant in a first solid callus growth medium containing glucose for a period of time sufficient for phenolic secretion from the explant to enable undifferentiated callus to form the explant;

   c) transferring the callus, after the phenolic secretion has stopped, to a second growth medium containing sucrose;

   d) culturing the callus in the second callus growth medium for a period of of time sufficient to allow development of embryogenic callus providing at least one embryo or embryogenic clumps;

   e) transferring the embryo or embryogenic clumps to a plant germination medium; and

   f) culturing the embryo or embryogenic clumps on the plant germination medium for a period of time sufficient to develop a plantlet from the embryo or embryogenic clumps with the proviso that cultivars Coker 201 and 315 are excluded.

2. The method according to claim 1 in which the explant in step a) is selected from the group consisting of hypocotyl, cotyledon and mixtures thereof derived from a cotton seedling and immature zygotic embryos.

3. The method according to claim 1 or 2 in which the explant is developed by:

   (i) sterilizing the seed in a first sterilizing solution;

   (it) rinsing the seed with sterile water;

   (iii) sterilizing the seed in a second sterilizing solution;

(iv) rinsing the second sterilization medium from the seed with sterile water;

(v) transferring the seed to a seed germination medium; and

(vi) growing the seed in the seed germination medium in the dark for a period of time sufficient to produce a seedling; and

(vii) excising the explant from the seedling.

4. The method according to claim 3 in which the first sterilizing solution is an aqueous solution containing 95% by volume ethanol and the second sterilizing solution is an aqueous solution containing 15% by weight sodium hypochlorite.

5. The method according to claim 3 or 4 in which the seed germination medium in (v) of claim 3 is a basic agar medium and growth prior to explant removal is for a period of up to 1 days.

6. The method according to any one of the previous claims in which the first growth medium in step b) is a full or half-strength Murashige and Skoog growth medium.

7. The method according to claim 6 in which the Murashige and Skoog growth medium contains naphthaleneacetic acid.

8. The method according to claim 7 in which the Murashige and Skoog growth medium further contains thiamine hydrochloride, kinetin and inositol.

9. The method according to claim 8 in which the Murashige and Skoog growth medium contains 0.4 mg/ml thiamine hydrochloride, 30g/l glucose, 2 mg/l naphthaleneacetic acid, 1 mg/l kinetin and 100 mg/l inositol.

10. The method according to any one of the previous claims in which the explant is cultured in step b) at a temperature of from 25 to 35°C under a daylight cycle of 16 hours of light and 8 hours of darkness.

11. The method according to claim 10 in which the light intensity during the hours of light is 2000 to 4000 lux.

12. The method according to any one of the previous claims in which the explant is cultured in the first growth medium during a period of phenolic secretions from the developing callus with the medium changed at least every 10 days.

13. The method according to any one of the previous claims in which the explant is cultured for three to four weeks.

14. The method according to any one of the previous claims in which the second growth medium in step c) is a full or half-strength Murashige and Skoog growth medium.

15. The method according to claim 14 in which the Murashige and Skoog growth medium contains naphthaleneacetic acid.

16. The method according to claim 15 in which the Murashige and Skoog growth medium further contains a cytokinin.

17. The method according to claim 15 or 16 in which the Murashige and Skoog growth medium contains from 1 to 10 mg/l naphthaleneacetic acid and from 0 to 1 mg/l cytokinin.

18. The method according to any one of the previous claims in which the callus is cultured in step d) at a temperature from 25 to 35°C under a daylight cycle of 16 hours of light and 8 hours of darkness.

19. The method according to claim 18 in which the light intensity during the hours of light is 2000 to 4000 lux.

20. The method according to claim 18 or 19 in which the callus is cultured for a period of time sufficient to form yellow to white embryogenic callus.

21. The method according to any one of the previous claims in which the embryogenic callus in step d) is subdivided and suspended in the second growth medium to form a suspension culture and said callus is grown to form embryogenic clumps of at least 600 µm in size.

22. The method according to claim 21 in which the suspension culture at the beginning of growth contains from 750 to 1000 mg of callus parts per 8 ml second growth medium.

23. The method according to claim 21 in which embryogenic clumps of a size greater than 600 µm are filtered out.

24. The method according to any one of claims 21 to 23 in which the embryogenic clumps of a size less than 600 µm are resuspended in fresh second growth medium for further growth of embryogenic clumps.

25. The method according to any one of claims 21 to 24 in which embryogenic clumps greater than 800 µm are removed from the suspension for plantlet formation.

26. The method according to any one of claims 1 to 25 in which the plant germination medium in step e) contains a source of ammonium.

27. The method according to any one of claims 1 to 25 in which the plant germination medium in step e) is a Beasley and Ting's medium, rich in a source of nitrogen.

28. The method according to claim 27 in which the Beasley and Ting's medium contains up to 500 mg/l casein hydrolysate and up to 1200 mg/l ammonium nitrate.

29. The method according to any one of claims 1 to 28 in which the embryo or embryogenic clumps in step f) are cultured for a period of 2 to 3 weeks.

30. The method according to any one of claims 1 to 29 in which the plantlet in step f) is transferred to the soil under conditions of hight humidity for a time sufficient to mature the plant to enable transfer to a hot house and then the field for growth to final maturity.


**Patentansprüche**

1. Verfahren für die Regeneration einer Baumwollpflanze aus somatischen Zellen, welches die Schritte umfaßt:

   a) zur Verfügung stellen eines Baumwollexplantates;

   b) Kultivieren des Explantates in einem ersten festen Kalluswachstumsmedium, welches Glucose enthält, für eine Zeitspanne, die ausreichend ist für phenolische Sekretion aus dem Explantat, um undifferenzierten Kallus in die Lage zu versetzen, das Explantat zu bilden;

   c) Transferieren des Kallus in ein zweites Wachstumsmedium, das Saccharose enthält, nachdem die phenolische Sekretion beendet ist;

   d) Kultivieren des Kallus in dem zweiten Kalluswachstumsmedium für eine Zeitspanne, die ausreichend ist, um Entwicklung von embryogenem Kallus zu erlauben, wobei mindestens ein Embryo oder embryogener Zellhaufen zur Verfügung gestellt wird;

   e) Transferieren des Embryos oder embryogenenen Zellhaufens auf ein Pflanzenkeimungsmedium; und

   f) Kultivieren des Embryos oder embryogenen Zellhaufens auf dem Pflanzenkeimungsmedium für eine Zeitspanne, die ausreichend ist, um ein Pflänzchen aus dem Embryo oder embryogenen Zellhaufen zu entwickeln, unter der Voraussetzung, daß Kultivare Coker 201 und 315 ausgeschlossen sind.

2. Verfahren gemäß Anspruch 1 in welchem das Explantat in Schritt a) ausgewählt ist aus der Gruppe, bestehend aus Hypokotyl, Kotyledon und Mischungen davon, welche von einem Baumwollkeimling und unreifen zygotären Embryos abstammen.

3.  Verfahren gemäß Anspruch 1 oder 2, in welchem das Explantat entwickelt wird durch:

    (i) Sterilisieren des Samens in einer ersten Sterilisationslösung;

    (ii) Abspülen des Samens mit sterilem Wasser;

    (iii) Sterilisieren des Samens in einer zweiten Sterilisationslösung;

    (iv) Abspülen des zweiten Sterilisationsmediums von dem Samen mit sterilem Wasser;

    (v) Transferieren des Samens auf ein Samenkeimungsmedium; und

    (vi) Heranwachsen des Samens in dem Samenkeimungsmedium im Dunkeln für eine Zeitspanne, die ausreichend ist, um einen Keimling herzustellen, und

    (vii) Ausschneiden des Explantates von dem Keimling.

4.  Verfahren gemäß Anspruch 3, in welchem die erste Sterilisationslösung eine wäßrige Lösung ist, die 95% in Volumen Ethanol enthält und die zweite Sterilisationslösung eine wäßrige Lösung ist, die 15% in Gewicht Natriumhypochlorit enthält.

5.  Verfahren gemäß Ansprurch 3 oder 4, in welchem das Samenkeimungsmedium in (v) von Anspruch 3 ein basisches Agarmedium ist und Wachstum vor Entfernen des Explantates für eine Periode von bis zu 14 Tagen andauert.

6.  Verfahren gemäß mindestens einem der vorhergehenden Ansprüche, in welchem das erste Wachstumsmedium in Schritt b) komplettes oder halb konzentriertes Murashige und Skoog Wachstumsmedium ist.

7.  Verfahren gemäß Anspruch 6, in welchem das Murashige und Skoog Wachstumsmedium Naphthalinessigsäure enthält.

8.  Verfahren gemäß Anspruch 7, in welchem das Murashige und Skoog Wachstumsmedium weiter Thiaminhydrochlorid, Kinetin und Inositol enthält.

9.  Verfahren gemäß Anspruch 8, in welchem das Murashige und Skoog Wachstumsmedium 0,4 mg/ml Thiaminhydrochlorid, 30 g/l Glucose, 2 mg/l Naphthalinessigsäure, 1 mg/l Kinetin und 100 mg/l Inositol enthält.

10. Verfahren gemäß mindestens einem der vorhergehenden Ansprüche, in welchem das Explantat in Schritt b) bei einer Temperatur von 25 bis 35°C unter einem Tageslichtzyklus von 16 Stunden an Licht und 8 Stunden an Dunkelheit kultiviert wird.

11. Verfahren gemäß Anspruch 10, in welchem die Lichtintensität während der Stunden an Licht 2000 bis 4000 Lux ist.

12. Verfahren gemäß mindestens einem der vorhergehenden Ansprüche, in welchem das Explantat in dem ersten Wachstumsmedium während einer Periode an phenolischen Sekretionen aus dem sich entwickelnden Kallus kultiviert wird, wobei das Medium mindestens alle 10 Tage ausgetauscht wird.

13. Verfahren gemäß mindestens einem der vorhergehenden Ansprüche, in welchem das Explantat für drei bis vier Wochen kultiviert wird.

14. Verfahren gemäß mindestens einem der vorhergehenden Ansprüche, in welchem das zweite Wachstumsmedium in Schritt c) ein komplettes oder halb konzentriertes Murashige und Skoog Wachstumsmedium ist.

15. Verfahren gemäß Anspruch 14, in welchem das Murashige und Skoog Wachstumsmedium Naphthalinessigsäure enthält.

16. Verfahren gemäß Anspruch 15, in welchem das Murashige und Skoog Wachstumsmedium weiter ein Cytokinin enthält.

**17.** Verfahren gemäß Anspruch 15 oder 16, in welchem das Murashige und Skoog Wachstumsmedium von 1 bis 10 mg/l Naphthalinessigsäure und von 0 bis 1 mg/l Cytokinin enthält.

**18.** Verfahren gemäß mindestens einem der vorhergehenden Ansprüche, in welchem der Kallus in Schritt d) bei einer Temperatur von 25 bis 35°C unter einem Tageslichtzyklus von 16 Stunden an Licht und 8 Stunden an Dunkelheit kultiviert wird.

**19.** Verfahren gemäß Anspruch 18, in welchem die Lichtintensität während der Stunden an Licht 2000 bis 4000 Lux ist.

**20.** Verfahren gemäß Anspruch 18 oder 19, in welchem der Kallus für eine Zeitspanne kultiviert wird, die ausreichend ist, um gelben bis weißen embryogenen Kallus zu bilden.

**21.** Verfahren gemäß mindestens einem der vorhergehenden Ansprüche, in welchem der embryogene Kallus in Schritt d) unterteilt wird und in dem zweiten Wachstumsmedium suspendiert wird, um eine Suspensionskultur zu bilden und der Kallus herangewachsen wird, um embryogene Zellhaufen von einer Größe von mindestens 600 um zu bilden.

**22.** Verfahren gemäß Anspruch 21, in welchem die Suspensionskultur zu Beginn des Wachstums von 750 bis 1000 mg an Kallusteilen pro 8 ml zweitem Wachstumsmedium enthält.

**23.** Verfahren gemäß Anspruch 21, in welchem embryogene Zellhaufen von einer Größe größer als 600 um herausgefiltert werden.

**24.** Verfahren gemäß mindestens einem der Ansprüche 21 bis 23, in welchem die embryogenen Zellhaufen von einer Größe weniger als 600 um zum weiteren Wachstum von embryogenen Zellhaufen in frischem zweiten Wachstumsmedium resuspendiert werden.

**25.** Verfahren gemäß mindestens einem der Ansprüche 21 bis 24, in welchem embryogene Zellhaufen größer als 800 um zur Bildung von Pflänzchen aus der Suspension entfernt werden.

**26.** Verfahren gemäß mindestens einem der Ansprüche 1 bis 25, in welchem das Pflanzenkeimungsmedium in Schritt e) eine Quelle an Ammonium enthält.

**27.** Verfahren gemäß mindestens einem der Ansprüche 1 bis 25, in welchem das Pflanzenkeimungsmedium in Schritt e) ein Beasley und Ting's Medium ist, welches reich an einer Stickstoffquelle ist.

**28.** Verfahren gemäß Anspruch 27, in welchem das Beasley und Ting's Medium bis zu 500 mg/l Casein-Hydrolysat und bis zu 1200 mg/l Ammoniumnitrat enthält.

**29.** Verfahren gemäß mindestens einem der Ansprüche 1 bis 28, in welchem der Embryo oder embryogene Zellhaufen in Schritt f) für eine Periode von 2 bis 3 Wochen kultiviert werden.

**30.** Verfahren gemäß mindestens einem der Ansprüche 1 bis 29, in welchem das Pflänzchen in Schritt f) unter Bedingungen von hoher Luftfeuchtigkeit in die Erde transferiert wird, für eine Zeit, die ausreichend ist, um die Pflanze zu reifen, um Transfer in ein Warm haus und dann aufs Feld zum Wachstum bis zur endgültigen Reife zu ermöglichen.

## Revendications

**1.** Procédé pour la régénération d'un cotonnier à partir de cellules somatiques, qui comprend les étapes consistant à:

a) obtenir un explant de coton;
b) mettre en culture l'explant dans un premier milieu de croissance de cal solide contenant du glucose pendant une durée suffisante pour que la sécrétion phénolique à partir de l'explant permette au cal indifférencié de former l'explant;
c) transférer le cal, après l'arrêt de la sécrétion phénolique, dans un deuxième milieu de croissance contenant du saccharose;

d) mettre en culture le cal dans le deuxième milieu de croissance de cal pendant une durée suffisante pour permettre le développement d'un cal embryogénique donnant au moins un embryon ou des amas embryogéniques;

d) transférer l'embryon ou les amas embryogéniques dans un milieu de germination des plantes; et

f) mettre en culture l'embryon ou les amas embryogéniques sur le milieu de germination des plantes pendant une durée suffisante pour développer une plantule à partir de l'embryon ou des amas embryogéniques à condition que les cultivars Coker 201 et 315 soient exclus.

2. Procédé selon la revendication 1, dans lequel l'explant de l'étape a) est choisi dans le groupe constitué par l'hypocotyle, le cotylédon et leurs mélanges, dérivés d'un plant de coton et d'embryons zygotiques immatures.

3. Procédé selon la revendication 1 ou 2, dans lequel l'explant est développé:

(i) en stérilisant la graine dans une première solution de stérilisation;
(ii) en rinçant la graine avec de l'eau stérile;
(iii) en stérilisant la graine dans une deuxième solution de stérilisation;
(iv) en rinçant la graine avec de l'eau stérile pour éliminer le deuxième milieu de stérilisation ;
(v) en transférant la graine dans un milieu de germination des graines; et
(vi) en faisant pousser la graine dans le milieu de germination des graines à l'obscurité pendant une durée suffisante pour produire un plant; et
(vii) en excisant l'explant à partir du plant.

4. Procédé selon la revendication 3, dans lequel la première solution de stérilisation est une solution aqueuse contenant 95% en volume d'éthanol et la deuxième solution de stérilisation est une solution aqueuse contenant 15% en poids d'hypochlorite de sodium.

5. Procédé selon la revendication 3 ou 4, dans lequel le milieu de germination des graines en (v) de la revendication 3 est un milieu gélosé basique et la croissance avant le retrait de l'explant se fait pendant une période allant jusqu'à 14 jours.

6. Procédé selon l'une quelconque des revendications précédentes, dans lequel le premier milieu de croissance dans l'étape b) est un milieu de croissance de Murashige et Skoog complet ou à 50%.

7. Procédé selon la revendication 6, dans lequel le milieu de croissance de Murashige et Skoog contient de l'acide naphtalèneacétique.

8. Procédé selon la revendication 7, dans lequel le milieu de croissance de Murashige et Skoog contient en outre du chlorhydrate de thiamine, de la kinétine et de l'inositol.

9. Procédé selon la revendication 8, dans lequel le milieu de croissance de Murashige et Skoog contient 0,4 mg/ml de chlorhydrate de thiamine, 30 g/l de glucose, 2 mg/l d'acide naphtalèneacétique, 1 mg/l de kinétine et 100 mg/l d'inositol.

10. Procédé selon l'une quelconque des revendications précédentes, dans lequel l'explant est cultivé dans l'étape b) à une température de 25 à 35°C sous un cycle de lumière du jour de 16 heures de lumière et de 8 heures d'obscurité.

11. Procédé selon la revendication 10, dans lequel l'intensité de la lumière durant les heures de lumière est de 2000 à 4000 lux.

12. Procédé selon l'une quelconque des revendications précédentes, dans lequel l'explant est cultivé dans le premier milieu de croissance durant une période de sécrétions phénoliques à partir du cal qui se développe, le milieu étant changé au moins tous les 10 jours.

13. Procédé selon l'une quelconque des revendications précédentes, dans lequel l'explant est cultivé pendant trois à quatre semaines.

14. Procédé selon l'une quelconque des revendications précédentes, dans lequel le deuxième milieu de croissance dans l'étape c) est un milieu de croissance de Murashige et Skoog complet ou à 50%.

**15.** Procédé selon la revendication 14, dans lequel le milieu de croissance de Murashige et Skoog contient de l'acide naphtalèneacétique.

**16.** Procédé selon la revendication 15, dans lequel le milieu de croissance de Murashige et Skoog contient en outre une cytokinine.

**17.** Procédé selon la revendication 15 ou 16, dans lequel le milieu de croissance de Murashige et Skoog contient de 1 à 10 mg/l d'acide naphtalèneacétique et de 0 à 1 mg/l de cytokinine.

**18.** Procédé selon l'une quelconque des revendications précédentes, dans lequel le cal est cultivé dans l'étape d) à une température de 25 à 35°C sous un cycle de lumière du jour de 16 heures de lumière et de 8 heures d'obscurité.

**19.** Procédé selon la revendication 18, dans lequel l'intensité de la lumière durant les heures de lumière est de 2000 à 4000 lux.

**20.** Procédé selon la revendication 18 ou 19, dans lequel le cal est cultivé pendant une durée suffisante pour former un cal embryogénique qui va du jaune au blanc.

**21.** Procédé selon l'une quelconque des revendications précédentes, dans lequel le cal embryogénique de l'étape d) est subdivisé et mis en suspension dans le deuxième milieu de croissance pour former une culture en suspension et ledit cal est mis à croître pour former des amas embryogéniques ayant une taille d'au moins 600 μm.

**22.** Procédé selon la revendication 21, dans lequel la culture en suspension contient, au début de la croissance, de 750 à 1000 mg de parties de cal pour 8 ml de deuxième milieu de croissance.

**23.** Procédé selon la revendication 21, dans lequel les amas embryogéniques de taille supérieure à 600 μm sont séparés par filtration.

**24.** Procédé selon l'une quelconque des revendications 21 à 23, dans lequel les amas embryogéniques de taille inférieure à 600 μm sont remis en suspension dans du deuxième milieu de croissance frais pour permettre une croissance plus poussée des amas embryogéniques.

**25.** Procédé selon l'une quelconque des revendications 21 à 24, dans lequel les amas embryogéniques supérieurs à 800 μm sont enlevés de la suspension pour la formation de plantules.

**26.** Procédé selon l'une quelconque des revendications 1 à 25, dans lequel le milieu de germination des plantes dans l'étape e) contient une source d'ammonium.

**27.** Procédé selon l'une quelconque des revendications 1 à 25, dans lequel le milieu de germination des plantes dans l'étape e) est un milieu de Beasley et Ting, riche en source d'azote.

**28.** Procédé selon la revendication 27, dans lequel le milieu de Beasley et Ting contient jusqu'à 500 mg/l d'hydrolysat de caséine et jusqu'à 1200 mg/l de nitrate d'ammonium.

**29.** Procédé selon l'une quelconque des revendications 1 à 28, dans lequel l'embryon ou les amas embryogéniques de l'étape f) sont cultivés pendant une période de 2 à 3 semaines.

**30.** Procédé selon l'une quelconque des revendications 1 à 29, dans lequel la plantule de l'étape f) est transférée dans le sol dans des conditions d'humidité élevée pendant une durée suffisante pour que la plante se développe afin de permettre son transfert dans une serre, puis dans le champ en vue de sa croissance jusqu'à la maturité finale.

*Fig. 1*

## Fig.2

*Fig. 3*

# Fig. 4

*Fig. 5*

*Fig. 6*

*Fig. 7*

16                    16

*Fig. 8*

*Fig.9*

*Fig.10*

*Fig. 11*

*Fig. 12*

*Fig. 13*

*Fig. 14*

_Fig.15_

*Fig. 16*

Fig.17

*Fig.18.*

*Fig.19*

*Fig.20*

Fig.21

*Fig.22*

# Fig. 23

*Fig. 24*

*Fig. 25*

Fig. 26

# Fig.21

Fig. 28

Fig. 29

Fig. 30

# Fig. 31

Fig. 32

# *Fig.* 33

Eco R1

Nos/NPT/TK

pBr322

DEI PEP 10

pRK290

Tetr

## *Fig.34*

## *Fig.35*